# EUROPÄISCHE PATENTSCHRIFT

(11) **EP 0 783 555 B1**
(45) Veröffentlichungstag und Bekanntmachung des Hinweises auf die Patenterteilung: **11.12.2002**
(21) Anmeldenummer: 95933436.8
(22) Anmeldetag: 28.09.1995
(51) Int. Cl.: C09K 11/06, H05B 33/14, C08G 61/00

(54) **POLY(PARAPHENYLENVINYLEN)-DERIVATE UND IHRE VERWENDUNG ALS ELEKTROLUMINESZENZMATERIALIEN**
POLY(PARAPHENYLENE VINYLENE) DERIVATIVES AND THEIR USE AS ELECTROLUMINESCENT MATERIALS
DERIVES DE POLY(PARAPHENYLENE-VINYLENE) ET LEUR UTILISATION COMME MATERIAUX ELECTROLUMINESCENTS

(30) Priorität: 30.09.1994 DE 4435047; 17.02.1995 DE 19505416
(43) Veröffentlichungstag der Anmeldung: 16.07.1997
(73) Patentinhaber: Covion Organic Semiconductors GmbH, 65926 Frankfurt (DE)
(72) Erfinder: KREUDER, Willi, D-55126 Mainz (DE); LUPO, Donald, D-60316 Frankfurt (DE); SALBECK, Josef, D-65779 Kelkheim (DE); SCHENK, Hermann, D-65719 Hofheim (DE); STEHLIN, Thomas, D-65830 Kriftel (DE); HÖRHOLD, Hans-Heinrich, D-07747 Jena (DE); LUX, Andrea, D-07749 Jena (DE); TEUSCHEL, Annett, D-07747 Jena (DE); WIEDUWILT, Martina, D-07743 Jena (DE)
(74) Vertreter: Luderschmidt, Schüler & Partner GbR
(86) Internationale Anmeldenummer: EP9503835
(87) Internationale Veröffentlichungsnummer: WO96010617

(56) Entgegenhaltungen:
- EP-A- 0 502 202
- WO-A-90/13148
- WO-A-92/03490
- WO-A-92/03491
- WO-A-93/14177
- WO-A-94/20589
- WO-A-94/29883
- DD-A- 84 272
- Makromol.Chem., Macromol.Symp., Band 12, S.229-258 (1987), Hörhold und Helbig

## Beschreibung

Es besteht ein hoher industrieller Bedarf an großflächigen Festkörper-Lichtquellen für eine Reihe von Anwendungen, überwiegend im Bereich von Anzeigeelementen, der Bildschirmtechnologie und der Beleuchtungstechnik. Die an diese Lichtquellen gestellten Anforderungen können zur Zeit von keiner der bestehenden Technologien völlig befriedigend gelöst werden.

Als Alternative zu herkömmlichen Anzeige- und Beleuchtungselementen, wie Glühlampen, Gasentladungslampen und nicht selbstleuchtenden Flüssigkristallanzeigeelementen, sind bereits seit einiger Zeit Elektrolumineszenz(EL)materialien und -vorrichtungen, wie lichtemittierende Dioden (LED), in Gebrauch.

Neben anorganischen sind seit etwa 30 Jahren auch niedermolekulare organische Elektrolumineszenzmaterialien und -vorrichtungen bekannt (siehe z.B.
US-A-3,172,862). Bis vor kurzem waren aber solche Vorrichtungen in ihrer praktischen Verwendbarkeit stark eingeschränkt.

In WO 90/13148 und EP-A 0 443 861 sind Elektrolumineszenzvorrichtungen beschrieben, die einen Film aus einem konjugierten Polymer als lichtemittierende Schicht (Halbleiterschicht) enthalten. Solche Vorrichtungen bieten zahlreiche Vorteile, wie die Möglichkeit, großflächige, flexible Displays einfach und kostengünstig herzustellen. Im Gegensatz zu Flüssigkristalldisplays sind Elektrolumineszenzdisplays selbstleuchtend und benötigen daher keine zusätzliche rückwärtige Beleuchtungsquelle.

Eine typische Vorrichtung nach WO 90/13148 enthält eine lichtemittierende Schicht in Form eines dünnen, dichten Polymerfilms (Halbleiterschicht), der wenigstens ein konjugiertes Polymer enthält. Eine erste Kontaktschicht steht in Kontakt mit einer ersten Oberfläche, eine zweite Kontaktschicht mit einer weiteren Oberfläche der Halbleiterschicht. Der Polymerfilm der Halbleiterschicht hat eine genügend geringe Konzentration von extrinsischen Ladungsträgern, so daß beim Anlegen eines elektrischen Feldes zwischen den beiden Kontaktschichten Ladungsträger in die Halbleiterschicht eingebracht werden, wobei die eine Kontaktschicht positiv gegenüber der anderen wird, und die Halbleiterschicht Strahlung aussendet. Die in solchen Vorrichtungen verwendeten Polymere sind im allgemeinen konjugiert. Unter konjugiertem Polymer versteht man ein Polymer, das ein delokalisiertes Elektronensystem entlang der Hauptkette besitzt. Das delokalisierte Elektronensystem verleiht dem Polymer Halbleitereigenschaften und gibt ihm die Möglichkeit, positive und/oder negative Ladungsträger mit hoher Mobilität zu transportieren.

In WO 90/13148 wird als polymeres Material für die lichtemittierende Schicht PoIy(p-phenylenvinylen)(PPV) verwendet, und es wird vorgeschlagen, die Phenylgruppe in einem solchen Material durch ein heterocyclisches oder ein kondensiertes carbocyclisches Ringsystem zu ersetzen. Um eine bessere Stabilisierung gegenüber Sauerstoff, Licht und Temperatureinflüsse zu erreichen, wurden Derivate des PPV synthetisiert, in denen die Wasserstoffe der Vinylgruppen durch Phenylgruppen substituiert sind (H. H. Hörhold et al., A novel approach to light emitting polyarylenes: Cyclization of poly(arylene vinylenes), International Conference on Science and Technology of Synthetic Metals ICSM 94, 24.-29.7.1994, Seoul, Korea; A. V. Vannikov, A. C. Saidov, Mendeleev Commun. 1993, 54). Die Fluoreszenzquantenausbeute ist jedoch noch unbefriedigend.

Weiterhin sind monocyanosubstituierte PPV-Derivate bekannt (siehe z.B. N. C. Greenham et al., Nature 1993, 365, 628).

Obwohl mit diesen Materialien gute Ergebnisse erzielt wurden, sind beispielsweise die Lebensdauer, Photostabilität und die Stabilität gegen Luft und Wasser noch unbefriedigend. Weiterhin ist es mit den bisher bekannten Polymeren kaum möglich, eine blaue oder weiße Emission zu erzeugen.

Da zudem die Entwicklung von Elektrolumineszenzmaterialien, insbesondere auf Grundlage von Polymeren, noch in keiner Weise als abgeschlossen betrachtet werden kann, sind die Hersteller von Beleuchtungs- und Anzeigevorrichtungen an den unterschiedlichsten Elektrolumineszenzmaterialien für solche Vorrichtungen interessiert.

Dies liegt unter anderem auch daran, weil erst das Zusammenwirken der Elektrolumineszenzmaterialien mit den weiteren Bauteilen der Vorrichtungen Rückschlüsse auf die Qualität auch des Elektrolumineszenzmaterials zuläßt.

Aufgabe der vorliegenden Erfindung war es daher, neue Elektrolumineszenzmaterialien bereitzustellen, die bei Verwendung in Beleuchtungsoder Anzeigevorrichtung geeignet sind, das Eigenschaftsprofil dieser Vorrichtungen zu verbessern.

Es wurde nun überraschend gefunden, daß bestimmte, an der Vinylgruppe monoarylsubstituierte PPV-Derivate in besonderer Weise zur Verwendung als Elektrolumineszenzmaterialien geeignet sind.

Gegenstand der Erfindung ist daher die Verwendung von Polymeren, enthaltend Struktureinheiten der allgemeinen Formel (I),

-[A¹-(A²)C = CH-A³-CH = C(A²)]- (I)

wobei A¹, A² und A³ gleich oder verschieden ein- und/oder mehrkernige, gegebenenfalls über eine oder mehrere, vorzugsweise eine Brücke verknüpfte, und/oder kondensierte Aryl- und/oder Heteroarylgruppen sind, die gegebenenfalls substituiert sein können, und wobei von A¹ und A³ je zwei und von A² je eine Bindung ausgeht,
als Elektrolumineszenzmaterialien.

Die erfindungsgemäß verwendeten Polymere zeichnen sich vor allem durch hohe Stabilität bei hoher Fluoreszenzquantenausbeute aus.

Als Elektrolumineszenzmaterial im Sinne der Erfindung gelten Stoffe, die als aktive Schicht in einer Elektrolumineszenzvorrichtung Verwendung finden können.
Aktive Schicht bedeutet, daß die Schicht befähigt ist, bei Anlegen eines elektrischen Feldes Licht abzustrahlen (lichtemittierende Schicht) und/oder daß sie die Injektion und/oder den Transport der positiven und/oder negativen Ladungen verbessert (Ladungsinjektions- oder Ladungstransportschicht).

Gegenstand der Erfindung ist daher auch ein Elektrolumineszenzmaterial, enthaltend ein oder mehrere Polymere, enthaltend Struktureinheiten der Formel (I).

Polymer bedeutet im Sinne der Erfindung eine Verbindung, deren Elektrolumineszenzspektrum bei Anfügen weiterer Struktureinheiten im wesentlichen gleich bleibt.

Die erfindungsgemäß verwendeten Polymere weisen im allgemeinen 2 bis 1000, vorzugsweise 3 bis 500, besonders bevorzugt 4 bis 300, Struktureinheiten auf.

Bevorzugt sind weiterhin solche Polymere, bei denen die Symbole in der allgemeinen Formel (I) folgende Bedeutung haben:
- A¹, A³:: sind gleich oder verschieden 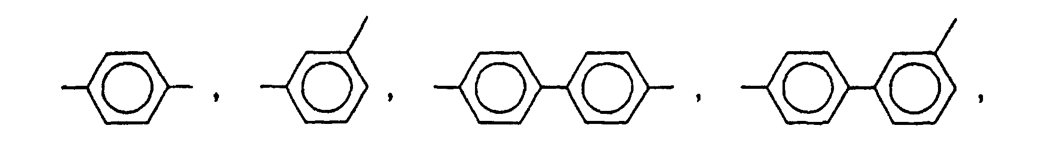 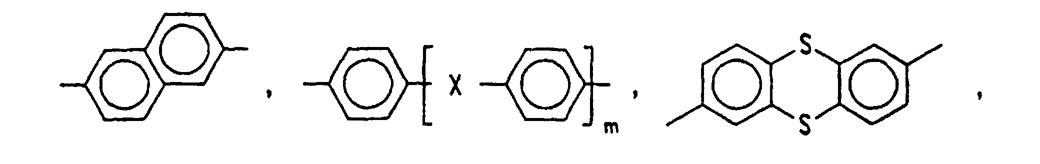 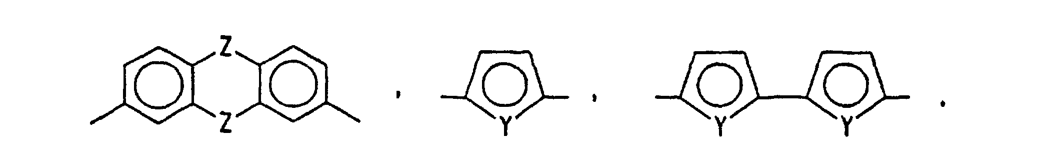 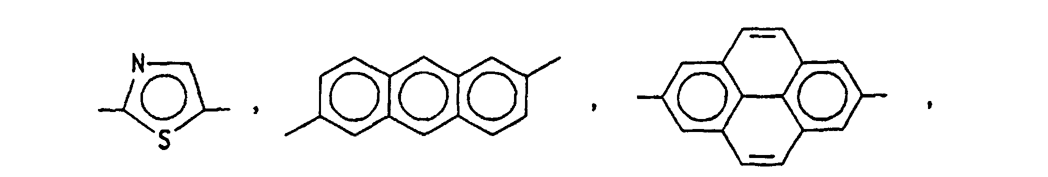 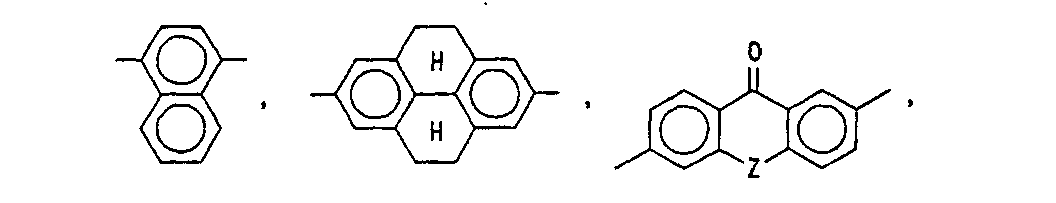 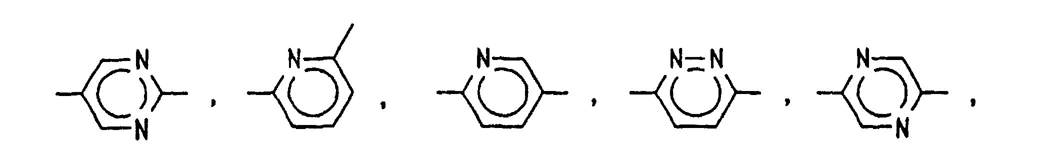 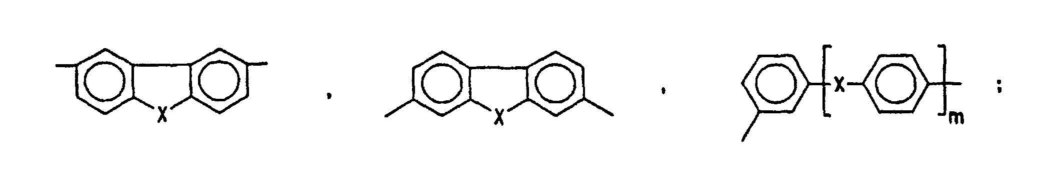 mit m = 1 bis 20, vorzugsweise 1, 2 oder 3, besonders bevorzugt 1, vorzugsweise ist nur für A³ m > 1;
A²: hat, gleich oder verschieden von A¹ und A³, die gleichen Bedeutungen wie A¹ und A³, wobei von den beiden möglichen Bindungsstellen zum Polymer jeweils nur eine realisiert ist;
A¹, A² und A³ können dabei unabhängig voneinander mit einem oder mehreren Resten R substituiert sein;
X: eine Einfachbindung, -O-, -S-, -SO-, -SO₂-, -CRR-, -CR = CR-, -CH₂-CH₂- oder -CHR-CHR-;
Y: -O-, -S- oder -NR'-;
Z: gleich oder verschieden -O- oder -S-;
R: gleich oder verschieden H, eine Alkylgruppe mit 1 bis 12 Kohlenstoffatomen, wobei auch eine oder zwei nicht benachbarte CH₂-Gruppen durch -O-, -S-, -CO-, -CO-O-, -O-OC- oder -Si(CH₃)₂- ersetzt sein können, -CF₃, -Ph, -O-Ph, -S-Ph, -SO-Ph, -SO₂-Ph, F, Cl, Br, I oder -CN;
R': H ,eine Alkylgruppe mit 1 bis 12 Kohlenstoffatomen oder -Ph.

Besonders bevorzugt haben die Symbole in der Formel (I) folgende Bedeutungen:
- A¹, A³:: sind gleich oder verschieden 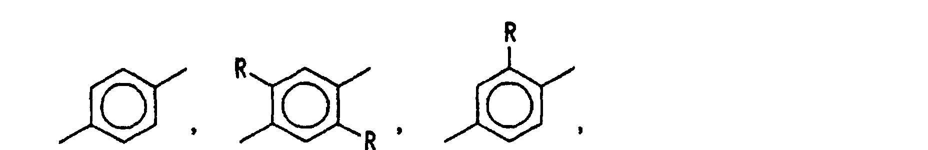 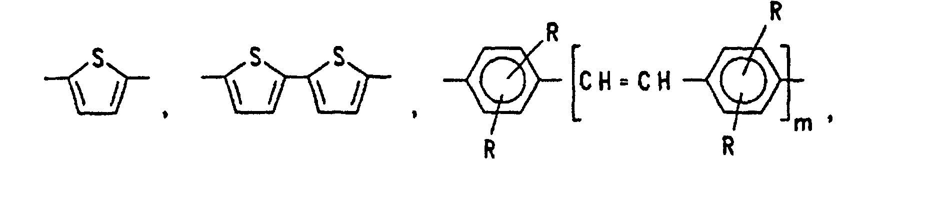 mit m = 1 bis 20, vorzugsweise 1, 2 oder 3, besonders bevorzugt 1, R ist vorzugsweise H, vorzugsweise ist nur für A³ m > 1; 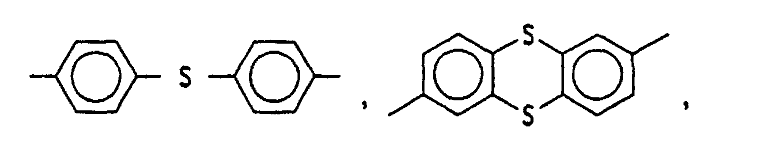 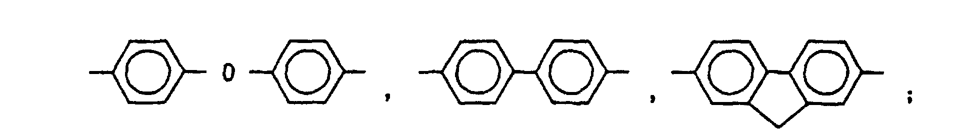
- A²:: hat, gleich oder verschieden von A¹ und A³, die gleichen Bedeutungen wie A¹ und A³, wobei von den beiden möglichen Bindungsstellen zum Polymer jeweils nur eine realisiert ist, oder ist

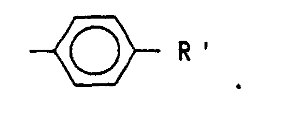

Ganz besonders bevorzugt sind folgende Gruppen von Polymeren, enthaltend Struktureinheiten der allgemeinen Formel (I): 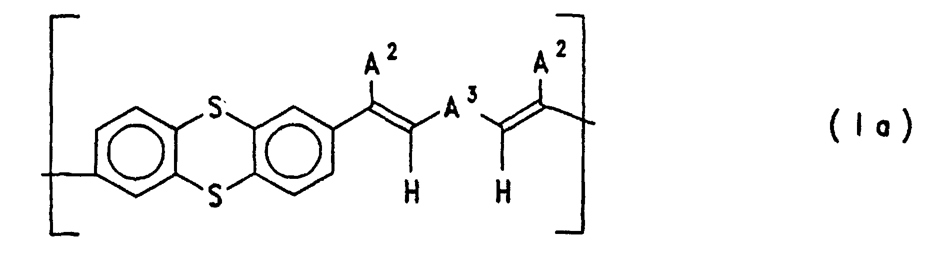 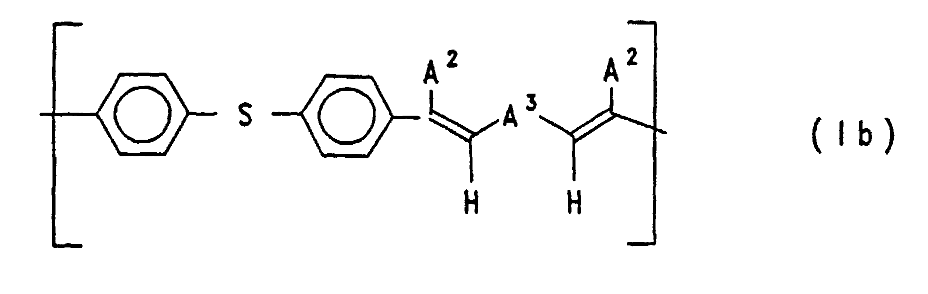 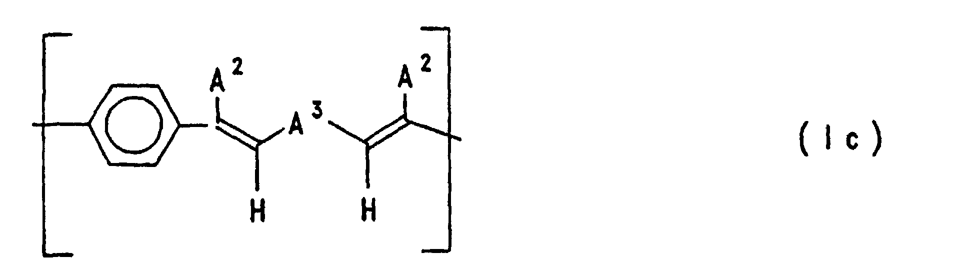 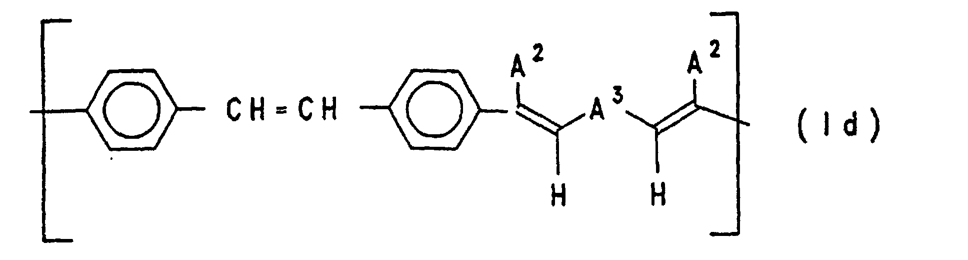 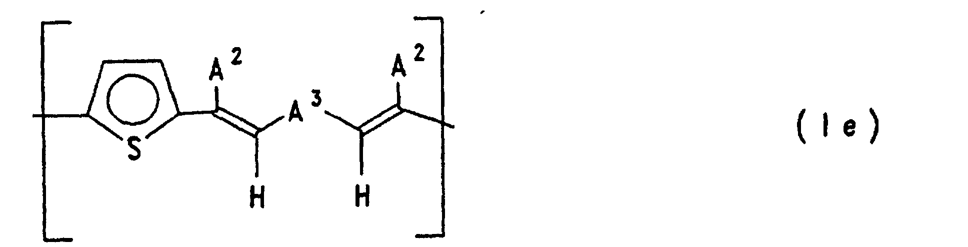 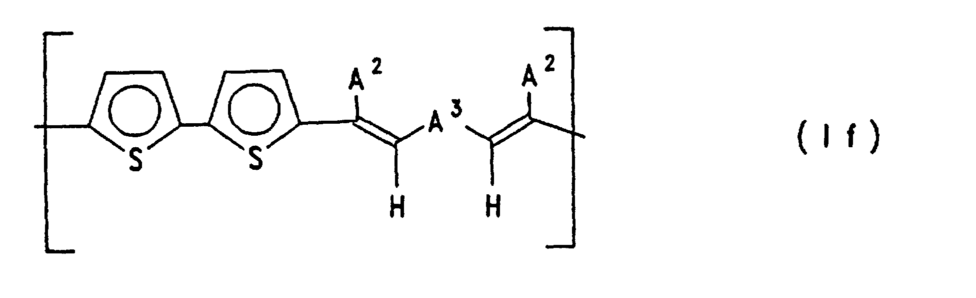 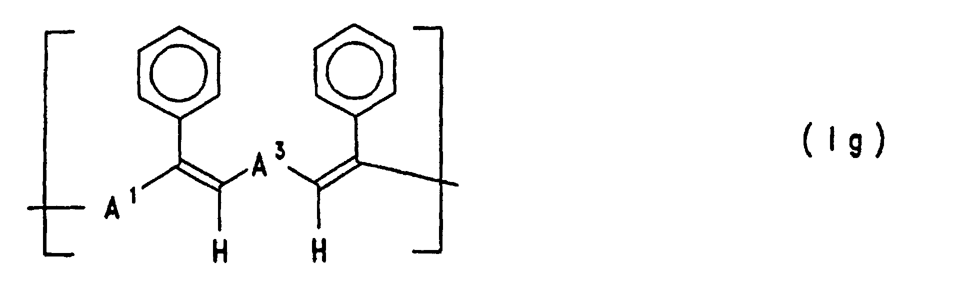 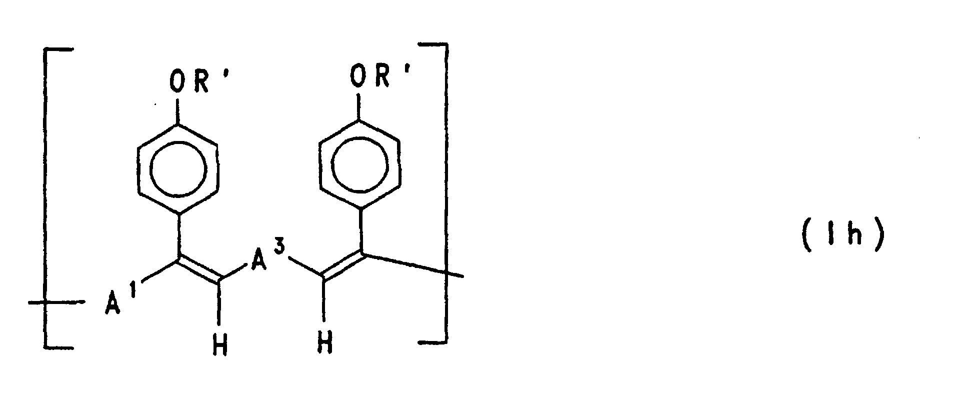 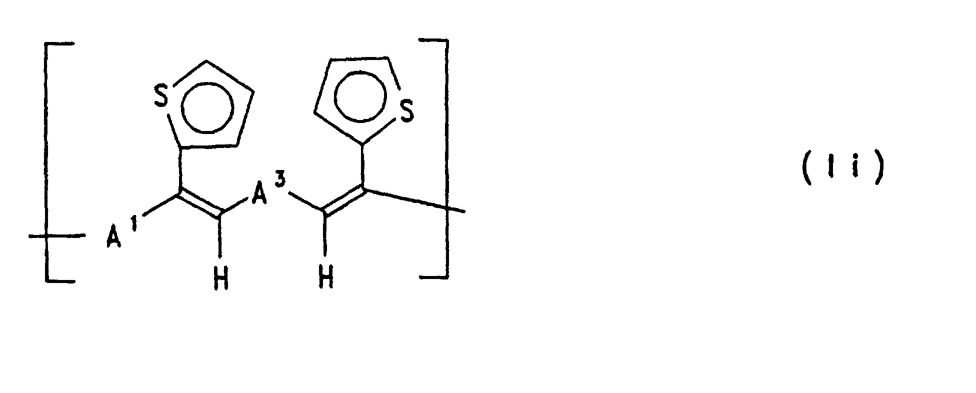 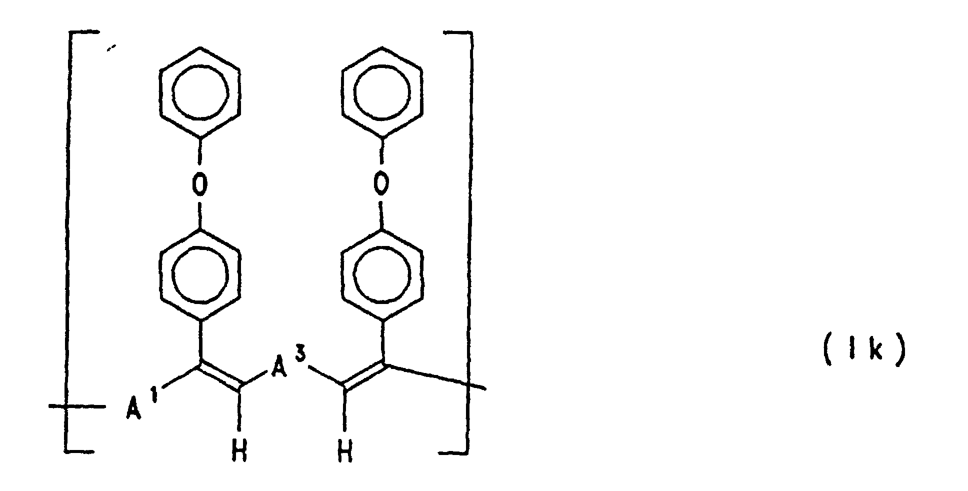 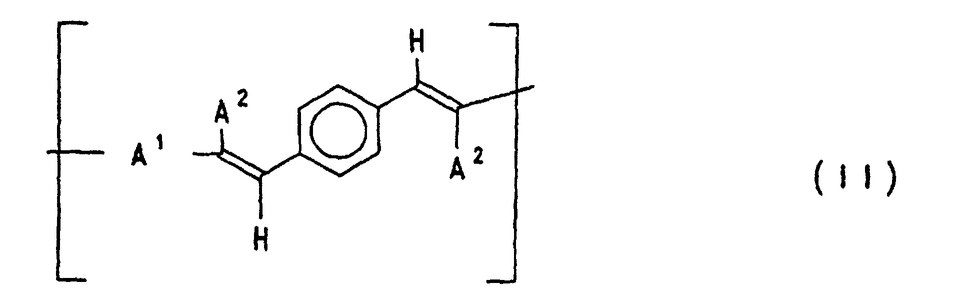 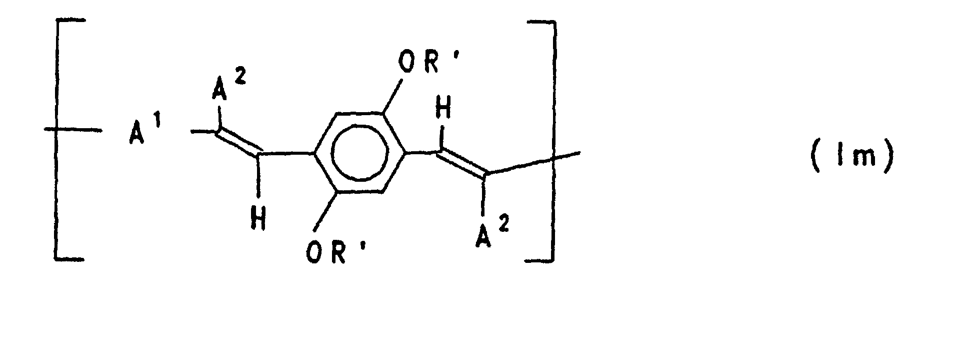 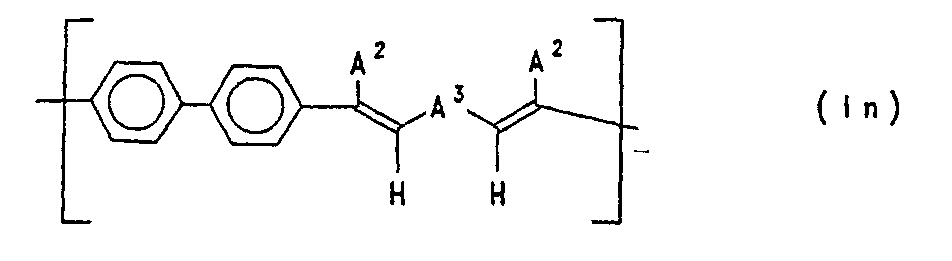 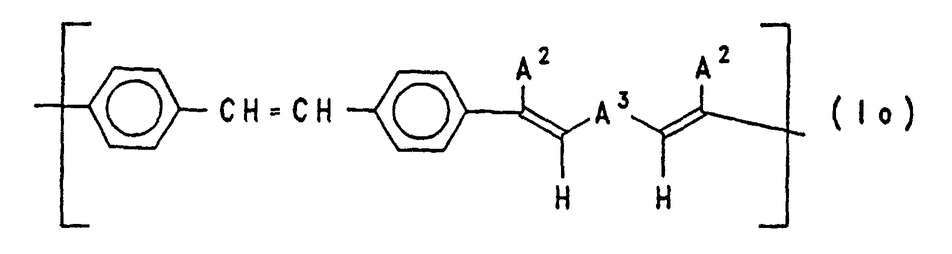 wobei A¹, A², A³ und R' die in Formel (I) angegebenen Bedeutungen haben.

Die Polymere, enthaltend Struktureinheiten der Formel (I), sind teilweise bekannt und teilweise neu.

Gegenstand der Erfindung sind daher auch Polymere, enthaltend Struktureinheiten der Formel (I), wobei die Symbole die oben angegebenen Bedeutungen haben, mit der Maßgabe, daß eine der Gruppen A¹, A² und A³ ein Heterocyclus sein muß.

Die Herstellung der erfindungsgemäßen oder erfindungsgemäß verwendeten Polymere erfolgt zweckmäßigerweise durch Kondensation von Diketonen der Formel (II), 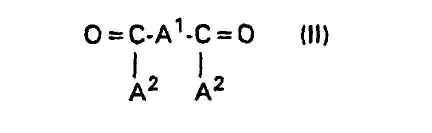 wobei A¹ und A² die in der Formel (I) angegebenen Bedeutungen haben, mit phosphororganischen Verbindungen der Formel (III) 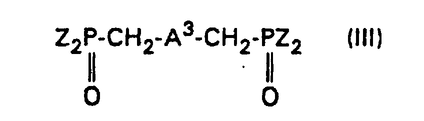 wobei A³ die in der Formel (I) angegebenen Bedeutungen besitzt und Z für Alkoxy-, vorzugsweise Ethoxy, bzw. Arylreste, vorzugsweise Phenyl, steht.

Die Kondensation erfolgt durch Einwirkung eines basischen Kondensationsmittels, vorzugsweise von Kalium-tert.-butylat.

Die Polykondensation wird zweckmäßigerweise so vorgenommen, daß man das äquimolare Gemisch der Ausgangskomponenten (I) und (III) in einem Lösungsmittel vorlegt und unter Inertgasatmosphäre und Rühren vorzugsweise mindestens molare Mengen Kondensationsmittel in Lösung oder Suspension einträgt.

Nach einer anderen Arbeitsvariante kann das Kondensationsmittel auch allein oder mit dem Diketon in einem Lösungsmittel vorgelegt und die Bisphosphorkomponente zugegeben werden. Als Lösungsmittel werden vorzugsweise Benzol, Toluol, Xylol oder Dimethylformamid verwendet, die Reaktionstemperatur beträgt vorzugsweise 60 bis 120°C und die Reaktionszeit 5 bis 20 Stunden. Die Umsetzungen erfolgen nahezu quantitativ.

Durch Zusatz von Wasser, gegebenenfalls einer Säure, wie Essigsäure, und Abtrennung der organischen Reaktionsphasen kann die Aufarbeitung erfolgen.
Die erhaltenen Kondensationsprodukte können zur Reinigung extrahiert, z.B. mit Alkoholen oder Essigsäure, oder aus einem Lösungsmittel in einem Nichtlösungsmittel gefällt werden.

Allgemein ist dieses Herstellungsverfahren beispielsweise in DD 84 272, Hörhold, H.-H.: Z. Chem.12, 41-52 (1972); Hörhold, H.-H.; Bergmann, R.; Gottschaldt, J.; Drefahl, G.: Acta Chim. Acad. Sci. Hung. 81, 239-251; Hörhold, H.-H.; Bergmann, R.: Advances in the Chemistry of Thermally Stable Polymers, Warszawa, Polish Scientific Publishers, 29-48 (1977); Hörhold, H.-H.; Helbig, M.: Makromol. Chem., Macromol. Symp. 12, 229-258 (1987) und Hörhold, H.-H.; Helbig, M.; Raabe, D.; Opfermann, J.; Scherf, U.; Stockmann, R.; Weiß, D.: Z. Chem. 27, 126 (1987) beschrieben.

Die Herstellung der Ausgangsverbindungen (II) und (III) erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart, beschrieben werden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch gemacht werden.

Die als Kondensationskomponenten benötigten Bis(diphenylphosphinoxide) bzw. Bis(phosphonsäureester) sind beispielsweise unter Anwendung der Michaelis-Arbusov-Reaktion aus den entsprechenden Bis(halogen-methyl)-Verbindungen mit Diphenylphosphinigsäureethylester (C₆H₅)₂P-O-C₂H₅ bzw. mit Triethylphosphit leicht zugänglich.

Die Synthese von aromatischen Diketonen kann beispielsweise durch die bekannte Friedel-Crafts-Reaktion, mit AlCl₃ als Katalysator, erfolgen, wie in Schema 1 an Hand von drei speziellen Verbindungen beispielhaft gezeigt ist.

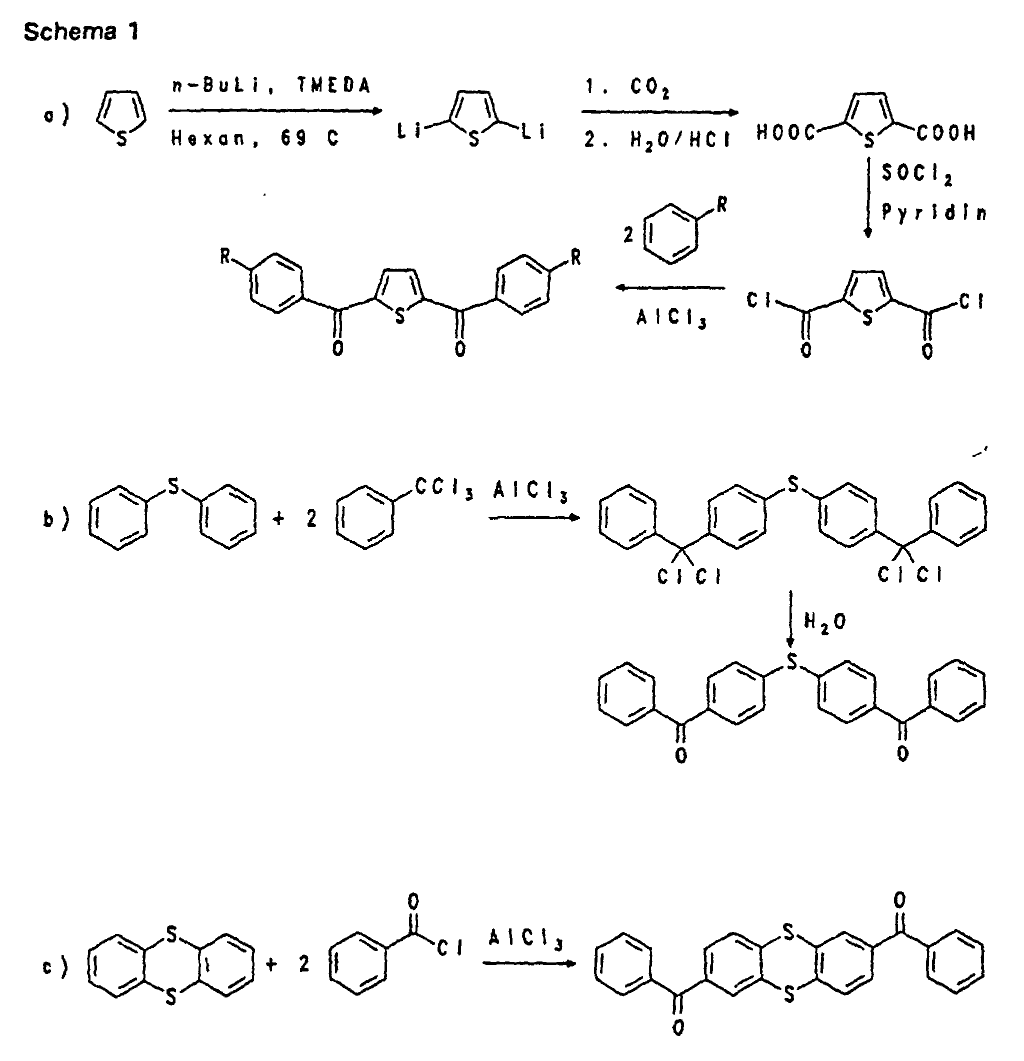

Eine weitere Variante ist die in Schema 2 beispielhaft dargestellte Grignardreaktion von Arylmagnesiumbromiden mit Dicyanoarylen:

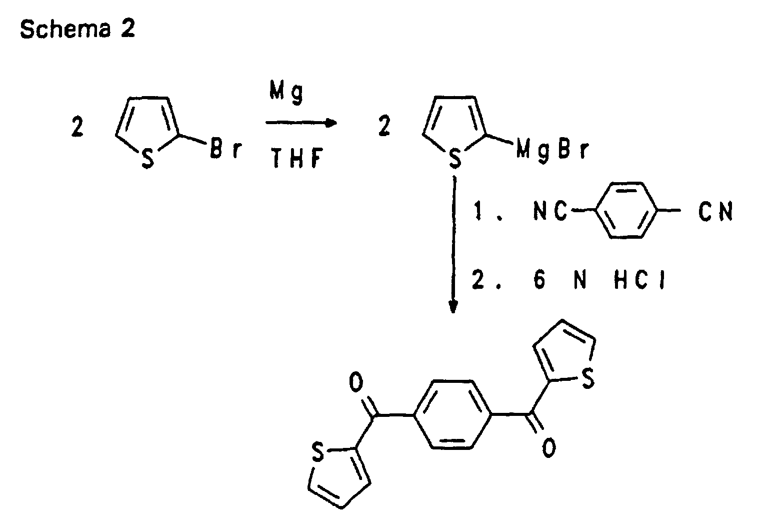

Bei der Horner-Reaktion können E/Z-Isomere entstehen. Isomere ergeben sich auch aus der möglichen Stellung der beiden Doppelbindungen zueinander (trans-transanti, trans-trans-syn, cis-trans-anti, cis-trans-syn, cis-cis-anti, cis-cis-syn); sie alle werden von der Erfindung umfaßt.

Durch den Einsatz unterschiedlicher Diketone und/oder Bisphosphonate werden in einfacher Weise Copolymere erhalten, die unterschiedliche Struktureinheiten der Formel (I) aufweisen. In solchen Copolymeren können die Reste R² in der Formel (I) gegebenenfalls auch unterschiedliche Bedeutungen haben.

Kleinere Gruppen von Polymeren, enthaltend Struktureinheiten der Formel (I), bei denen A¹ die Bedeutung -A'-A'- hat, wobei A' für einen elektronenreichen Aromaten steht, können alternativ auch oxidativ (z.B. mit FeCl₃, siehe u.a. P. Kovacic, N. B. Jones, Chem. Ber. 1987, 87, 357 bis 379; M. Weda, T. Abe, H. Awano, Macromolecules 1992, 25, 5125) oder elektrochemisch (siehe z.B. N. Saito, T. Kanbara, T. Sato, T. Yamamoto, Polym. Bull. 1993, 30, 285) polymerisiert werden.

Um als Elektrolumineszenzmaterialien Verwendung zu finden, werden die erfindungsgemäßen Polymere im allgemeinen nach bekannten, dem Fachmann geläufigen Methoden, wie Eintauchen (Dipping), Gießen (Casting) oder Spincoating, in Form eines Films auf ein Substrat aufgebracht.

Gegenstand der Erfindung ist daher auch ein Verfahren zur Herstellung eines Elektrolumineszenzmaterials, dadurch gekennzeichnet, daß man
a) eine phosphororganische Verbindung der Formel (III) 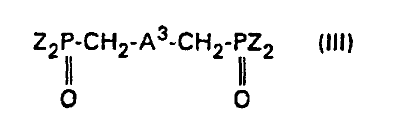 mit einem Diketon der Formel (II) 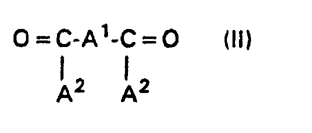 unter Einwirkung eines basischen Kondensationsmittels zu einem Polymer, enthaltend Struktureinheiten der Formel (I), kondensiert,

   -[A¹-(A²)C = CH-A³-CH = C(A²)]- (I)

   wobei A¹, A² und A³ gleich oder verschieden ein- und/oder mehrkernige, gegebenenfalls über eine oder mehrere, vorzugsweise eine Brücke verknüpfte, und/oder kondensierte Aryl- und/oder Heteroarylgruppen sind, die gegebenenfalls substituiert sein können, und wobei von A¹ und A³ je zwei und von A² je eine Bindung ausgeht; und
b) das so erhaltene Polymer in Form eines Films auf ein Substrat aufbringt.

Gegenstand der Erfindung ist weiterhin eine Elektrolumineszenzvorrichtung mit einer oder mehreren aktiven Schichten, wobei mindestens eine dieser aktiven Schichten ein oder mehrere erfindungsgemäße Polymere enthält. Die aktive Schicht kann beispielsweise eine lichtemittierende Schicht und/oder eine Transportschicht und/oder eine Ladungsinjektionsschicht sein.

Der allgemeine Aufbau solcher Elektrolumineszenzvorrichtungen ist beispielsweise in US 4,539,507 und US 5,151,629 beschrieben. Polymere enthaltende Elektrolumineszenzvorrichtungen sind beispielsweise in WO 90/13148 oder EP-A 0443861 beschrieben.

Sie enthalten üblicherweise eine elektrolumineszierende Schicht zwischen einer Kathode und einer Anode, wobei mindestens eine der Elektroden transparent ist. Zusätzlich kann zwischen der elektrolumineszierenden Schicht und der Kathode eine Elektroneninjektions- und/oder Elektronentransportschicht eingebracht sein und/oder zwischen der elektrolumineszierenden Schicht und der Anode eine Lochinjektions- und/oder Lochtransportschicht eingebracht sein. Als Kathode können z.B. Ca, Mg, Al, In, Mg/Ag dienen. Als Anode können z.B. Au oder ITO (Indiumoxid/Zinnoxid) auf einem transparentem Substrat, z.B. aus Glas oder einem transparenten Polymer, dienen.

Im Betrieb wird die Kathode auf negatives Potential gegenüber der Anode gesetzt. Dabei werden Elektronen von der Kathode in die Eiektroneninjektionsschicht/-Elektronentransportschicht bzw. direkt in die lichtemittierende Schicht injiziert. Gleichzeitig werden Löcher von der Anode in die Lochinjektionsschicht/Lochtransportschicht bzw. direkt in die lichtemittierende Schicht injiziert.

Die injizierten Ladungsträger bewegen sich unter dem Einfluß der angelegten Spannung durch die aktiven Schichten aufeinander zu. Dies führt an der Grenzfläche zwischen Ladungstransportschicht und lichtemittierender Schicht bzw. innerhalb der lichtemittierenden Schicht zu Elektronen/Loch-Paaren, die unter Aussendung von Licht rekombinieren.
Die Farbe des emittierten Lichtes kann durch die als lichtemittierende Schicht verwendete Verbindung variiert werden.

Elektrolumineszenzvorrichtungen finden Anwendung z.B. als selbstleuchtende Anzeigeelemente, wie Kontrollampen, alphanumerische Displays, Hinweisschilder, und in optoelektronischen Kopplern.

Die Erfindung wird durch die Beispiele näher erläutert, ohne sie dadurch beschränken zu wollen.

### Beispiele

Es bedeuten
- Tg:: Glasübergangstemperatur, gemessen mittels Differential-Scanning-Calorimetry (DSC)
- Mₙ:: Zahlenmittel des Molekulargewichts
- VPO:: Vapor Pressure Osmometry (siehe z.B. Cherdron, Kern, Braun, Praktikum der Makromolekularen Chemie)

### A. Ausgangsverbindungen

### 2,7-Dibenzoylthianthren

In einem 500 ml Mehrhalskolben mit Claisenaufsatz, Rührer, Rückflußkühler und Innenthermometer wird unter Kühlung mit Eis-Kochsalz-Mischung zu einer gerührten Suspension von 33,33 g AlCl₃ (0,25 mol) in 150 ml 1,2-Dichlorethan eine Lösung aus 21,63 g Thianthren (0,1 mol) und 46,91 g Benzotrichlorid (0,24 mol) in 75 ml 1,2-Dichlorethan so zugetropft, daß die Temperatur nicht über 5°C ansteigt. Dann wird ca. 6 Stunden bei Raumtemperatur gerührt und über Nacht stehen gelassen. Zur Vervollständigung der Umsetzung wird weitere 3 Stunden bei 35°C gerührt und anschließend mit Eis/HCl hydrolysiert. Das entstandene Tetrachlorid wird ca. 10 Stunden am Rückfluß mit 200 ml Eisessig und 15 ml Wasser zum Diketon hydrolysiert. Das Rohprodukt wird in Toluol aufgenommen, getrocknet und eingeengt. Es wird in Portionen zu 3 bis 4 g mit je 130 g Kieselgel 60 H chromatograpiert (Eluent: Toluol), wobei man auch insgesamt 0,4 g Monoketon isoliert. Das 2,7-Dibenzoylthianthren wird anschließend aus Toluol umkristallisiert. Man erhält hellgelbe Kristalle, m.p. 195°C.

| Ausbeute: 28,3 g = 67 % d. Theorie | | | |
|---|---|---|---|
| C₂₆H₁₆O₂S₂ | ber. C 73,56 | H 3,80 | S 15,10 |
| (424,508) | gef. C 73,66 | H 3,82 | S 15,19 |

### 4,4'-Dibenzoyldiphenylsulfid

In einem 250 ml Mehrhalskolben mit Rührer, Rückflußkühler und Tropftrichter, werden 26,5 g AlCl₃ (0,2 mol) in 100 ml 1,2-Dichlorethan vorgelegt. Zu dieser Suspension tropft man unter Rühren und Kühlen mit Eis-Kochsalz-Mischung eine Lösung aus 14,0 g Diphenylsulfid (0,075 mol) und 22,5 g Benzoylchlorid in 35 ml 1,2-Dichlorethan so zu, daß die Temperatur 5°C nicht übersteigt. Dann wird 6 Stunden bei Raumtemperatur weitergerührt und über Nacht stehen gelassen. Die eiskalte Reaktionsmischung wird zur Hydrolyse auf eine Eis/HCl-Mischung gegossen. Die organische Phase wird abgetrennt, zweimal mit verdünnter NaOH und mehrmals mit destilliertem Wasser gewaschen. Dann wird mittels Wasserdampfdestillation das Lösungsmittel entfernt und das Rohprodukt zweimal aus DMF/Ethanol = 1:1 umkristallisiert. Man erhält weiße, glänzende Blättchen, m.p. 173°C.

| Ausbeute: 15,9 g = 57 % d. Theorie | | | |
|---|---|---|---|
| C₂₆H₁₈O₂S | ber. C 79,19 | H 4,57 | S 8,12 |
| (394,462) | gef. C 79,39 | H 4,60 | S 8,11 |

### Hydrochinondioctylether

In einem 500 ml Mehrhalskolben mit Rührer, Rückflußkühler und Tropftrichter werden unter Argon 20 g Hydrochinon (0,18 mol) in 250 ml DMF vorgelegt und vorsichtig mit 8,6 g Natriumhydrid (0,36 mol) versetzt. Man erhitzt unter Rühren und Rückfluß, bis die Wasserstoffentwicklung beendet ist und tropft das n-Octylbromid innerhalb von 30 Minuten zu. Die Reaktionslösung wird 2 Stunden bei 130°C gerührt und über Nacht stehengelassen. Die Reaktionsmischung wird auf 60°C erwärmt, vom Bodensatz abfiltriert und eingeengt. Aus dem Rückstand erhält man nach zweimaligem Umkristallisieren aus Methanol weiße Schuppen, m.p. 56°C.

| Ausbeute: 48,3 g = 80 % d. Theorie | | |
|---|---|---|
| C₂₂H₃₈O₂ | ber. C 79,05 | H 11,38 |
| (334,23) | gef. C 79,42 | H 11,33 |

### 2,5-Dioctyloxy-1,4-bis(brommethyl)benzol

22 g Hydrochinondioctylether (0.066 mol), 27,8 g Paraformaldehyd (0,927 mol), 34,7 g Natriumbromid (0,337 mol) und 400 ml Eisessig werden in einem 1l Mehrhalskolben mit Rührer, Rückflußkühler und Tropftrichter unter Rühren auf 80°C erhitzt und innerhalb einer Stunde tropfenweise mit einer Mischung aus 35 ml konzentrierter Schwefelsäure und 45 ml Eisessig versetzt. Man läßt 5 Stunden bei 80°C rühren und dann auf Raumtemperatur abkühlen. Der abgeschiedene Feststoff wird abgesaugt und mehrmals mit Wasser gewaschen. Das organische Filtrat wird vorsichtig in 500 ml destilliertes Wasser gegossen und mehrmals mit Methylenchlorid extrahiert. Nach Vereinigung des Extrakts mit dem Feststoff wird über CaCl₂ getrocknet und anschließend das Lösungsmittels abdestilliert. Nach Umkristallisieren des Rückstandes aus Hexan erhält man weiße, verfilzte Kristalle, m.p. 83°C.

| Ausbeute: 26,6 g = 77 % d. Theorie | | | |
|---|---|---|---|
| C₂₄H₄₀Br₂O₂ | ber. C 55,40 | H 7,60 | Br 30,72 |
| (520,366) | gef. C 55,56 | H 7,69 | Br 30,08 |

### 2,5-Dioctyloxy-1,4-xylylen-bis(diethylphosphonat)

20,81 g 2,5-Dioctyloxy-1,4-bis(bromomethyl)benzol (0,04 mol) und 13,29 g Triethylphosphit (0,08 mol) werden in einem 250 ml Zweihalskolben mit Magnetrührer und Destillationsbrücke auf 130°C erhitzt, wobei entstehendes Ethylbromid abdestilliert. Innerhalb 1 Stunde wird auf 190°C erhitzt und noch 3 Stunden bei dieser Temperatur gerührt. Zur Entfernung restlichen Triethylphosphits wird bei dieser Temperatur noch 30 Minuten Vakuum angelegt. Nach Abkühlen erstarrt der Rückstand zu einer wachsartigen Masse, die aus Petrolether umkristallisiert wird. Man erhält feine, weiße Kristalle, m.p. 41°C.

| Ausbeute: 20,8 g = 82 % d. Theorie | | |
|---|---|---|
| C₃₂H₆₀O₈P₂ | ber. C 60,55 | H 9,53 |
| (634,745) | gef. C 60,50 | H 9,57 |

### B. Polymere

### Bsp. 1: Poly[2,5-dioctyloxy-1,4-phenylen-2-phenylvinylen-2,5-thienylen-1-phenylvinylen]

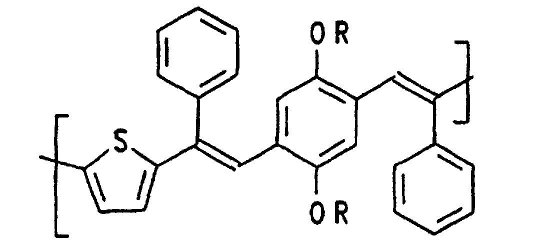

In einem mit Argon gespülten 350 ml Mehrhalskolben mit Innenthermometer, Rührer, Rückflußkühler und Tropftrichter werden 2,046 g 2,5-Dibenzoylthiophen (0,007 mol) und 4,443 g 2,5-Dioctyloxy-1,4-xylylen-bis(diethylphosphonat) (0,007 mol) in 150 ml Toluol, getrocknet über Na/Benzophenon, vorgelegt und auf 40 bis 60°C erwärmt, bis sich die Ausgangsstoffe vollständig gelöst haben. Nun gibt man 3,14 g Kalium-tert.-butylat (0,028 mol) zu und erhitzt die Reaktionsmischung 20 Stunden am Rückfluß. Dann läßt man auf Raumtemperatur abkühlen und hydrolysiert den Ansatz mit 100 ml 10 %iger Essigsäure unter mäßigem Rühren (Verhinderung der Emulsionsbildung). Die Toluolphase wird abgetrennt, dreimal mit destilliertem Wasser gewaschen und eingeengt. Der Rückstand wird zur Trocknung in Benzol aufgenommen und am Wasserabscheider erhitzt. Nach Filtration von ausgefallenen Salzen wird die Lösung auf 30 bis 40 ml eingeengt und in Methanol ausgefällt. Zur Reinigung der Probe wird der Vorgang einmal wiederholt. Der Reststoff wird abgesaugt, mit Methanol gewaschen und 20 Stunden im Vakuum (0,01 mm) getrocknet. Man erhält ein dunkelrotes, niedrigschmelzendes Pulver, Tg = 33°C.

| Ausbeute: 3,32 g = 77 % d. Theorie, Mₙ (VPO) = 4700 g.mol⁻¹ | | | |
|---|---|---|---|
| C₄₂H₅₀O₂S | ber. C 81,50 | H 8,14 | S 5,18 |
| (618,878) | gef. C 77,88 | H 8,35 | S 5,11 |

### Bsp. 2: Poly[2,5-dioctyloxy-1,4-phenylen-2-thienylvinylen-1,4-phenylen-1-thienylvinylen]

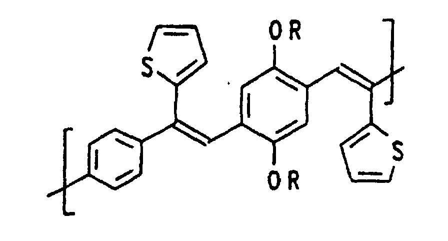

- Ansatz:: 2,089 g 1,4-Bis(2-thienoyl)benzol (0.007 mol)
4,443 g 2,5-Dioctyloxy-1,4-xylylen-bis(diethylphosphonat) (0,007 mol)
3,14 g Kalium-tert.-butylat (0,028 mol)
150 ml Toluol
Durchführung: analog Beispiel 1
Man isoliert ein ockerfarbenes, niedrigschmelzendes Pulver, Tg = 58°C.

| Ausbeute: 2,1 g = 48 % d. Theorie, Mₙ (VPO) = 12900 g.mol⁻¹ | | | |
|---|---|---|---|
| C₄₀H₄₈O₂S₂ | ber. C 76,88 | H 7,74 | S 10,26 |
| (624,902) | gef. C 75,09 | H 7,85 | S 9,06 |

### Bsp. 3: Poly[2,5-dioctyloxy-1,4-phenylen-2-phenylvinylen-1,4-phenylen-thio-1,4-phenylen-1-phenylvinylen]

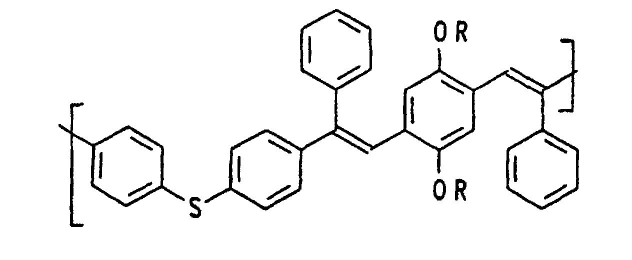

- Ansatz:: 2,761 g 4,4'-Dibenzoyldiphenylsulfid (0,007 mol)
4,443 g 2,5-Dioctyloxy-1,4-xylylen-bis(diethylphosphonat)
(0,007 mol)
3,14 g Kalium-tert.-butylat (0,028 mol)
150 ml Toluol
Durchführung: analog Beispiel 1
Man erhält ein leuchtend gelbes, niedrigschmelzendes Pulver, Tg = 54°C.

| Ausbeute: 3,69 g = 73 % d. Theorie, Mₙ (VPO) = 6300 g.mol⁻¹ | | | |
|---|---|---|---|
| C₅₀H₅₆O₂S | ber. C 83,29 | H 7,83 | S 4,45 |
| (721,006) | gef. C 81,52 | H 7,62 | S 4,12 |

### Bsp. 4: Poly[2,5-dioctyloxy-1,4-phenylen-2-phenylvinylen-2,7-thianthrenylen-1-phenyl-vinylen]

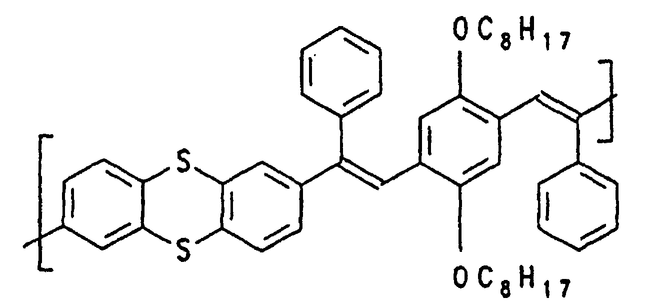

- Ansatz:: 2,972 g 2,7-Dibenzoylthianthren (0,007 mol)
4,443 g 2,5-Dioctyloxy-1,4-xylylen-bis(diethylphosphonat)
3,14 g Kalium-tert.-butylat (0,028 mol) 150 ml Toluol
Durchführung: analog Beispiel 1
Man erhält ein leuchtend gelbes Pulver, Tg = 78°C.

| Ausbeute: 3,80 g = 72 % d. Theorie, Mₙ (VPO) = 6500 g.mol⁻¹ | | | |
|---|---|---|---|
| C₅₀H₅₄O₂S₂ | ber. C 79,95 | H 7,25 | S 8,54 |
| (751,050) | gef. C 79,32 | H 7,19 | S 8,23 |

### Bsp. 5: Poly[1,4-phenylen-2-phenylvinylen-2,5-thienylen-1-phenylvinylen]

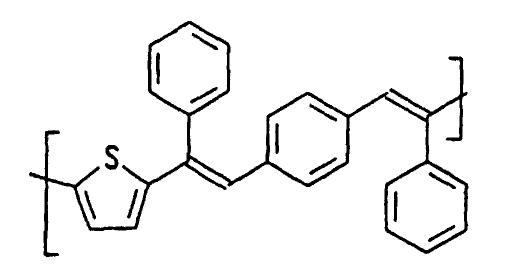

- Ansatz:: 5,846 g 2,5-Dibenzoylthiophen (0,02 mol)
7,566 g 1,4-Xylylen-bis(diethylphosphonat) (0,02 mol)
9,78 g Kalium-tert.-butylat (0,08 mol)
200 ml Toluol
Durchführung: analog Beispiel 1
Nach der Hydrolyse des Reaktionsgemisches verblieb ein unlöslicher Anteil, der getrocknet, pulverisiert und anschließend mit heißem Methanol extrahiert wurde. Das IR-Spektrum war völlig identisch mit dem des löslichen Anteils. Mit dem löslichen Anteil wurde in der gewohnten Weise verfahren, jedoch wurde die Substanz durch Fällen in Hexan isoliert. Eine Fraktionierung erfolgte durch Ausfällen der Verbindung aus Methylenchlorid in Methanol. Nach Extraktion des erhaltenen Feststoffes mit Methanol und Trocknung bei 60°C im Vakuum (0,05 Torr, 20 Stunden) erhält man ein organgefarbenes Pulver, Tg = 141°C.

| Ausbeute: löslicher Anteil: 3,32 g = 46 % d. Theorie, Mₙ (VPO) = 2300 g.mol⁻¹ unlöslicher Anteil: 1,38 g = 19 % d. Theorie | | | | |
|---|---|---|---|---|
| C₂₅H₁₈S | ber. C 86,15 | H 5,01 | S 8,84 | |
| (362,464) | gef. C 82,26 | H 5,36 | S 8,51 | (lösl. Anteil) |

### Bsp. 6: Poly[1,4-phenylen-2-(4-methoxyphenyl)vinylen-2,5-thienylen-1-(4-methoxy-phenyl)vlnylenl

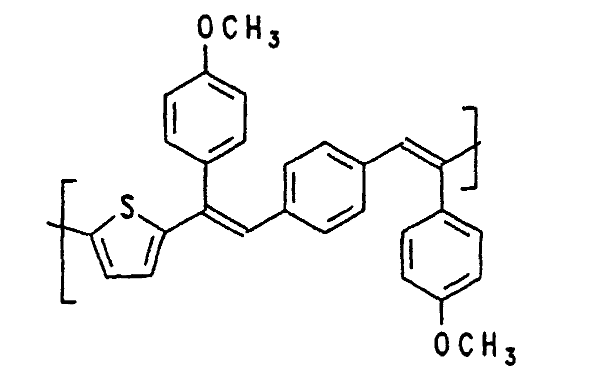

- Ansatz:: 3,524 g 2,5-Bis(4-methoxybenzoyl)thiophen (0,01 mol)
3,783 g 1,4-Xylylen-bis(diethylphosphonat) (0,01 mol)
4,89 g Kalium-tert.-butylat (0,04 mol) 150 ml Toluol
Durchführung: analog Beispiel 1 (lösl. Anteil)
Man erhält ein orangerotes Pulver, Tg = 147°C.

| Ausbeute: 2,65 g = 63 % d. Theorie, Mₙ (VPO) = 4800 g.mol⁻¹ | | | |
|---|---|---|---|
| C₂₈H₂₂O₂S | ber. C 79,59 | H 5,25 | S 7,59 |
| (422,514) | gef. C 78,30 | H 5,13 | S 7,62 |

### Bsp. 7: Poly[1,4-phenylen-2-(4-phenoxyphenyl)vinylen-2,5-thienylen-1-(4-phenoxyphenyl)vinylen]

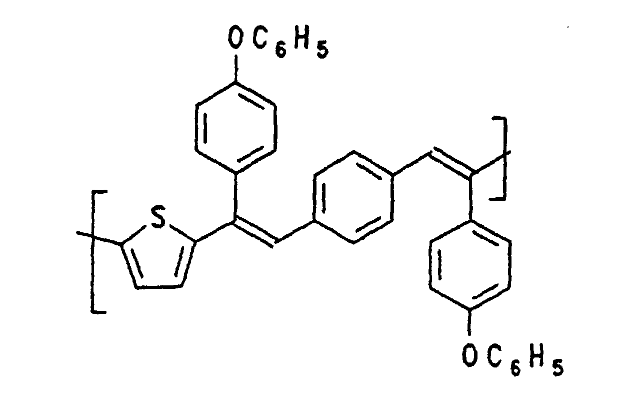

- Ansatz:: 4,765 g 2,5-Bis(4-phenoxybenzoyl)thiophen (0,01 mol)
3,783 g 1,4-Xylylen-bis(diethylphosphonat) (0,01 mol)
4,89 g Kalium-tert.-butylat (0,04 mol)
150 ml Toluol
Durchführung: analog Beispiel 1, jedoch erfolgte die Fraktionierung der Probe durch Fällen in Aceton.
Man erhält ein orangerotes Pulver, Tg = 133°C.

| Ausbeute: 3,01 g = 55 % d. Theorie, Mₙ (VPO) = 11300 g.mol⁻¹ | | | |
|---|---|---|---|
| C₃₈H₂₆O₂S | ber. C 83,49 | H 4,79 | S 5,86 |
| (546,646) | gef. C 82,09 | H 5,01 | S 5,72 |

### Bsp. 8: Poly[2,5-dioctyloxy-1,4-phenylen-2-phenylvinylen-2,2'-bithienylen-1-phenyl-vinyten]

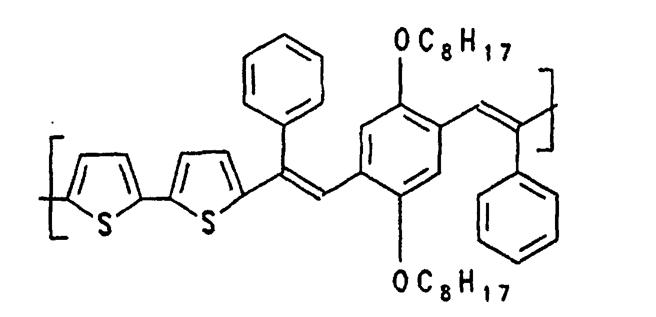

Zu einer gerührten Suspension von 3 g FeCl₃.6 H₂O (11,1 mmol) in 250 ml CH₂Cl₂ wird langsam eine Lösung von 0,75 g 2,5-Dioctyloxy-1,4-bis[2-phenyl-2-(2-thienyl)ethenyl]benzol 6b (1,06 mmol) zugetropft. Die Lösung färbt sich sofort tiefblau. Nach beendeter Zugabe wird die Mischung 3 Tage bei Raumtemperatur gerührt. Dann wird die Mischung mit verdünnter Salzsäure (150 ml H₂O, 5 ml konz. HCI) hydrolysiert, die Methylenchloridphase mehrmals mit destilliertem Wasser neutral gewaschen und eingeengt. Danach wird der Rückstand zur Trocknung in 150 ml Benzol aufgenommen und am Wasserabscheider erhitzt. Die Lösung wird auf ca. 100 ml eingeengt und über 0,5 g Kieselgel 60 H filtriert. Durch Zentrifugieren und Filtration erhält man eine klare Lösung, die auf ca. 15 ml eingeengt und in Methanol ausgefällt wird. Zur Reinigung wird der Fällungsvorgang einmal wiederholt. Das resultierende Pulver wird 20 Stunden im Vakuum
(0,05 Torr) bei 60°C getrocknet.

| Ausbeute: 0,59 g = 80 % d. Theorie, Mₙ (VPO) = 25000 g.mol⁻¹ | | | |
|---|---|---|---|
| C₄₆H₅₄O₂S₂ | ber. C 78,81 | H 7,48 | S 9,15 |
| (700,996) | gef. C 78,57 | H 7,64 | S 9,21 |

### Beispiel 9:

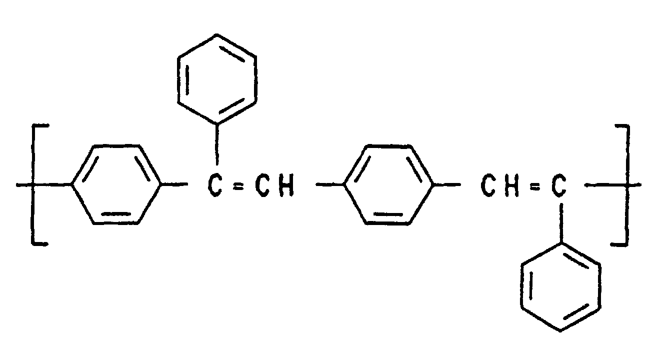

Zu einer Lösung von 0,02 mol Kalium-tert.-butylat in 75 ml trockenem Tofuot tropft man unter Inertgas und Rühren eine Lösung von 0,005 mol (1,4-Bis(benzoyl)-benzol (Terephthalophenon) und 0,005 mol p-Xylylen-bis(phosphonsäurediäthylester) in 75 ml trocknem Toluol bei einer Temperatur von 110°C. Nach 10 Stunden Reaktionsdauer wird mit verdünnter Essigsäure hydrolysiert und analog Beispiel 1 aufgearbeitet.
Die Ausbeute beträgt nach Reinigung 75 %.
Tg = 198,5°C, Mₙ = 6240 g/mol (VPO) nach Umfällen.

### Beispiel 10:

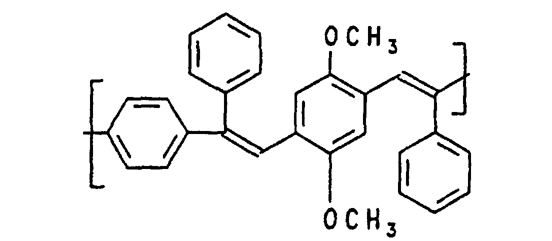

Zu 0,01 mol Terephthalophenon und 0,04 mol Kalium-tert.-butylat in 100 ml trockenem Toluol werden bei 100°C unter Rühren und Inertgas 0,01 mol 2,5-Dimethoxy-p-xylylen-bis(diphenylphosphinoxid)in 150 ml trocknem Toluol zugetropft. Reaktionszeit und Aufarbeitung wie im Beispiel 1.
Man erhält in 95 %iger Ausbeute ein chromgelbes Rohprodukt, das nach Extraktion im Bereich von 225 bis 240°C schmilzt und in Lösung starke gelbgrüne Fluoreszenz zeigt: Mₙ (VPO) = 5100 g/mol, T_{g} = 195°C.

### Bsp. 11: Poly[1,4-phenylen-2-phenylvinylen-4,4'-biphenylen-1-phenylvinylen]

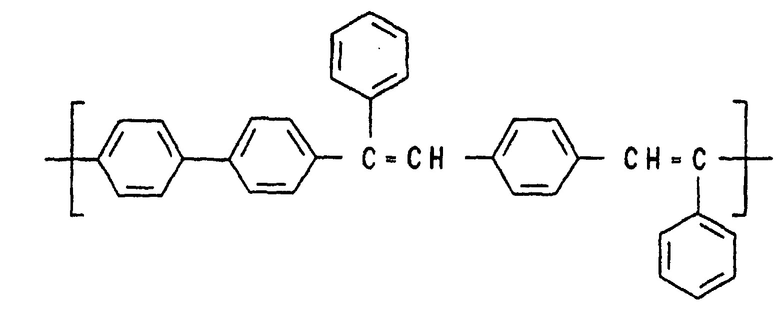

Die Herstellung erfolgt analog Beispiel 1.
Mₙ (VPO): 3650; Tg: 220°C

### Bsp. 12: Poly[2,5-dimethoxy-1,4-phenylen-2-(4-methoxyphenyl)vinylen-1,4-phenylen-1-(4-methoxyphenyl)vinylen]

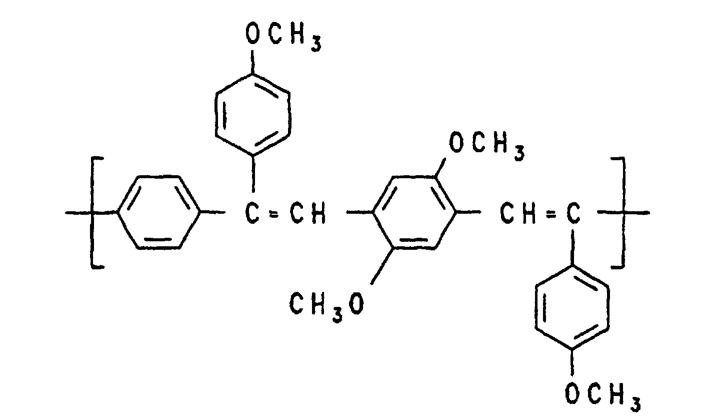

Die Herstellung erfolgt analog Beispiel 1.
Mₙ (VPO): 8750; Tg: 179,5°C

### Beispiel 13 Poly[1,4-phenylen-vinylen-1,4-phenylen-2-(4-phenoxyphenyl)-1,4-phenylen-1-(4-phenoxyphenyl)vinylen]

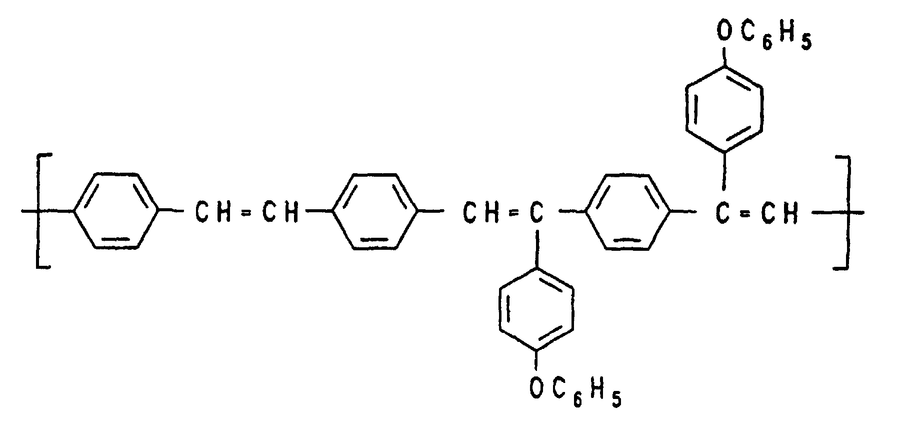

Die Herstellung erfolgt analog Beispiel 1.
Mₙ (VPO): 4780; Tg: 152°C

### Bsp. 14: Poly(2,5-dimethoxy-1,4-phenylen-[2-(4-fluorphenyl)-1,2-vinylen]-1,4-phenylen-[1-(4-fluorphenyl)-1,2-vinylen])

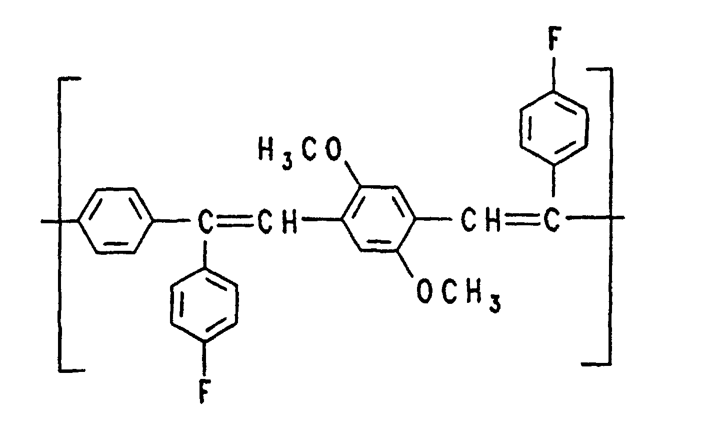

| | | |
|---|---|---|
| 1,4-Bis-(4-fluorbenzoyl)-benzol | 3,242 g | (10 mmol) |
| 2,5-Dimethoxy-p-xylylen-bis(diethylphosphonat) | 4,523 g | (10,3 mmol) |
| Kalium-tert.-butylat | 3,6 g | (32 mmol) |

In einem trockenen, mit Argon gespülten 500 ml Mehrhalskolben mit Rührer und Rückflußkühler, werden das Diketon und das Diphosphonat in rund 100 bis 150 ml frisch von Na/Benzophenon abdestilliertem Toluol gelöst, wobei die Reaktionsmischung auf 100 bis 110°C erhitzt wird. Dann wird vorsichtig unter Argon das Katium-tert.-butylat zugegeben, dabei schäumt die Reaktionsmischung auf und färbt sich rot-grün. Nach der Zugabe des Kalium-tert.-butylats wird 8 Stunden bei einer Badtemperatur von 120 bis 130°C gerührt. Nach Ablauf der Reaktionszeit wird der Ansatz mit 100 ml 10 %iger Essigsäure hydrolysiert. Man läßt über Nacht stehen und trennt dann die Phasen. Die organische Phase wird einbis zweimal mit Wasser gewaschen, und die wäßrige Phase wird mehrmals mit Toluol ausgerührt. Anschließend werden die organischen Phasen vereinigt und am Rotationsverdampfer bis zur Trockene eingeengt. Der Rückstand wird in 250 bis 300 ml Benzol aufgenommen und einen Tag am Wasserabscheider gekocht. Die Lösung wird danach von dem verbleibenden Rückstand abfiltriert und soweit am Rotationsverdampfer auf 20-30 ml eingeengt, daß die Lösung in 400 ml Methanol fällbar ist. Der leuchtendgelbe Niederschlag wird abgesaugt und im Vakuum bei 120°C getrocknet.
Die Polymerrohausbeute beträgt 3,75 g (83 % d. Th.) und Mn (GPC) 6910 g mol⁻¹. Das Polymer wird anschließend mit Methanol extrahiert. Der Glaspunkt T_{g} beträgt 202°C.

| | |
|---|---|
| Mₙ (VPO) | 13.800 g mol⁻¹ |
| Mₙ (GPC) | 11.100 g mol⁻¹ |
| M_{w} (GPC) | 92.200 g mol⁻¹ |
| M_{w}/Mₙ | 8.334 |

| | | | |
|---|---|---|---|
| C₃₀H₂₂F₂O₂ [452,48] | ber. C: 79,62 | H: 4,90 | |
| | gef. C: 78,33 | H: 4,92 | P: 0,0 % nicht nachweisbar |

### Bsp. 15: Poly[1,4-phenylen-oxo-1,4-phenylen-[1-(4-fluorphenyl)-1,2-vinylen]-2,5-dimethoxy-1,4-phenylen-[2-(4-fluorphenyl)-1,2-vinylen])

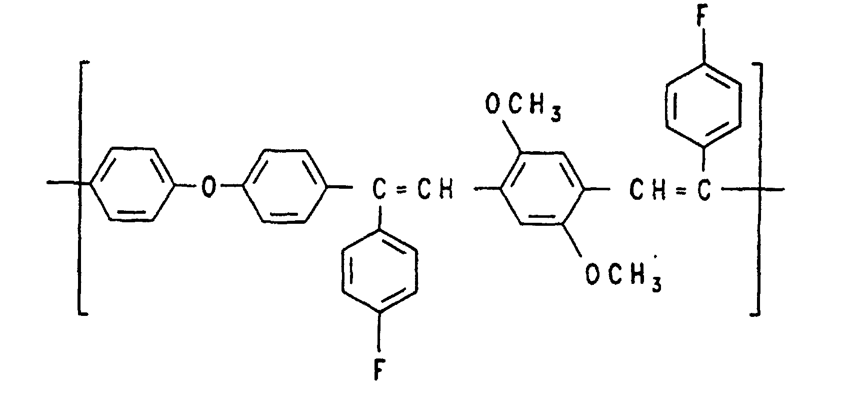

| | | |
|---|---|---|
| 1,4-Bis-(4-fluorbenzoyl)-diphenylether, | 4,144 g | (10 mmol) |
| 2,5-Dimethoxy-p-xylylen-bis(diethylphosphonat) | 4,384 g | (10,3 mmol) |
| Kalium-tert.-butylat | 3,6 g | (32 mmol) |

In einem trockenen, mit Argon gespülten 500 ml Mehrhalskolben mit Rührer und Rückflußkühler, werden das Diketon und das Diphosphonat in rund 100 bis 150 ml frisch von Na/Benzophenon abdestilliertem Toluol gelöst, wobei die Reaktionsmischung auf 100 bis 110°C erhitzt wird. Kalium-tert.-butylat wird zugegeben, wobei die Reaktionsmischung aufschäumt und sich dunkelorange färbt. Nach der Zugabe des Kalium-tert.-butylats wird 7 Stunden bei einer Badtemperatur von 120 bis 130°C gerührt. Nach Ablauf der Reaktionszeit wird der Ansatz mit 100 ml 10 %iger Essigsäure hydrolysiert. Man läßt über Nacht stehen und trennt dann die Phasen. Die organische Phase wird ein- bis zweimal mit Wasser gewaschen, und die wäßrige Phase wird mehrmals mit Toluol ausgerührt. Anschließend werden die organischen Phasen vereinigt und am Rotationsverdampfer bis zur Trockene eingeengt. Der Rückstand wird in 250 bis 300 ml Benzol aufgenommen und einen Tag am Wasserabscheider gekocht. Die Lösung wird danach von dem verbleibenden Rückstand abfiltriert und soweit am Rotationsverdampfer auf 20-30 ml eingeengt, daß die Lösung in 400 ml Methanol fällbar ist. Der leuchtendgelbe Niederschlag wird abgesaugt und nach Extrahieren mit Methanol im Vakuum bei 120°C getrocknet. Es werden 2,7 g (50 %) Rohpolymer erhalten.

Der Glaspunkt beträgt 187°C. Das Polymer ist in Methylenchlorid, Toluol und THF löslich.

| | |
|---|---|
| Mₙ(VPO) | 12.260 g mol⁻¹ |
| Mₙ (GPC) | 6.030 g mol⁻¹ |
| M_{w} (GPC) | 146. 000 g mol⁻¹ |

| | | | |
|---|---|---|---|
| C₃₈H₂₆F₂O₃ [544,57] | ber. C: 79,39 | H: 4,81 | |
| | gef. C: 77,22 | H: 5,10 | P: 0,0 % nicht nachweisbar |

### Bsp. 16: Poly(naphthalin-2,6-diyl-[1-phenyl-1,2-vinyl]-2,5-dimethoxy-1,4-phenylen-[2-phenyl-1,2-vinylen])

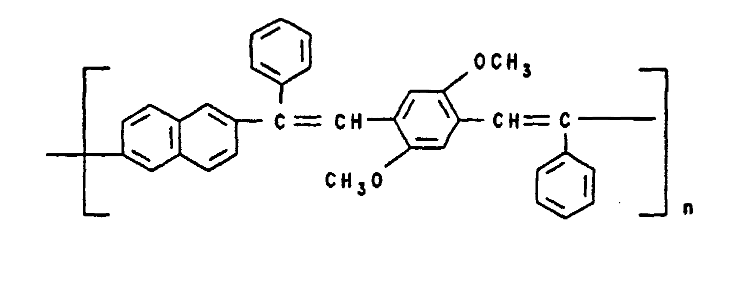

Die Herstellung erfolgt analog Beispiel 14.
Mₙ (VPO): 5900; T_{g} : 198°C

### Bsp. 17: Poly(napthalin-2,6-diyl-[1-(4-fluorphenyl)-1,2-vinylen]-2,5-dimethoxy-1,4-phenylen-[2,4-fluorphenyl-1,2-vinylen])

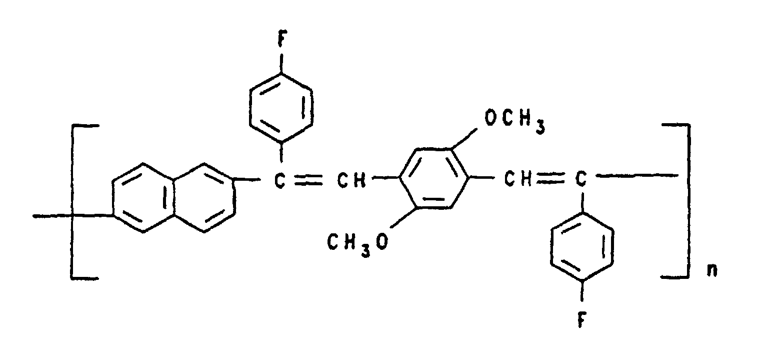

Die Herstellung erfolgt analog Beispiel 14.
Mₙ (VPO): 8000; T_{g} : 167°C

### Bsp. 18: Poly(thianthren-2,7-diyl-(1-phenyl-1,2-vinylen]-2,5-dimethoxy-1,4-phenylen-[2-phenyl-1,2-vinylen])

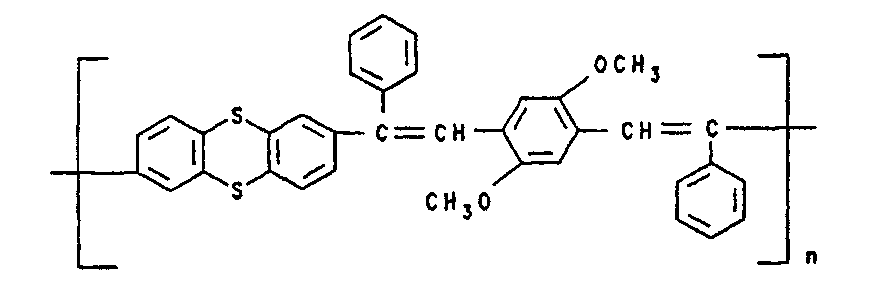

Die Herstellung erfolgt analog Beispiel 14.
Mₙ (GPC): 5600; M_{w} (GPC): 9080; T_{g} : 159°C

### Bsp. 19: Poly(pyridin-2,5-diyl)-[1-(4-fluorphenyl)-1,2-vinylen]-2,5-dimethoxy-1,4-phenylen-[2-(4-fluorphenyl)-1,2-vinylen])

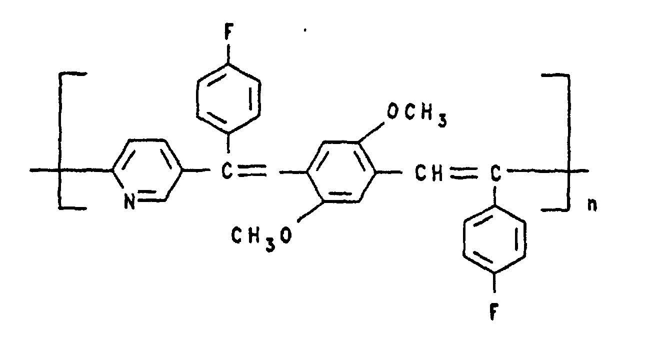

Die Herstellung erfolgt analog Beispiel 14.
Mn (GPC); 5300;
- λₘₐₓ :: 416 nm; Ig εₘₐₓ : 4,28
- λ_{01, max} :: 496 nm

### C. Elektrolumineszenzeigenschaften

In der Tabelle 1 sind Fluoreszenzquantenausbeuten und Oxidationspotentiale von erfindungsgemäßen Polymeren aufgeführt.

**Tabelle 1**

| Polymer gemäß Beispiel | Ø_{PL}¹⁾ | E^{0x1 2)} |
|---|---|---|
| 3 | 46 % | 1,05 |
| 4 | 56 % | 1,12 |
| 9 | 49 % | 1,26 |
| 11 | 54 % | 1,26 |
| 12 | 44 % | 0,95 |
| 13 | 49 % | 1,17 |
| 14 | 54 % | 1,07 |
| 15 | 33 % | 1,08 |

| | | |
|---|---|---|
| ¹⁾ Fluoreszenzquantenausbeute Ø_{PL} in CHCl₃-Lösung (siehe z.B. C. A. Parker, Photoluminescence of Solutions, Elsevier Publ. Comp., Amsterdam, 1968, S. 261 ff.) | | |
| ²⁾ Erstes Oxidationspotential bestimmt durch Cyclovoltammetrie an Pt-Elektrode gegen Ag/AgCl-Referenz in Dichlormethan, 0,1 m Tetrabutylammoniumhexafluorophosphat | | |

### Elektrolumineszenz-Vorrichtung

Eine Lösung des zu vermessenden Polymers in Chloroform 15 mg/ml wird unter Stickstoff durch Spin-Coating bei 1000 upm auf einen mit ITO (Indium-Zinn-Oxid) beschichteten Glasträger (strukturiert, Streifen 2 mm breit) aufgebracht. Der Glasträger wird über eine Schleuse unter Beibehaltung der Schutzgasatmosphäre in eine Hochvakuum-Bedampfungsanlage überführt. Bei 2x10⁻⁵ mbar werden quer zu den ITO-Streifen unter Verwendung einer Maske Ca-Streifen (2 mm breit, 230 nm dick) auf die Polymerschicht aufgedampft. Die so erhaltene Vorrichtung, ITO/Polymer/Ca, wird in einen Probenhalter gegeben und die Elektroden über Federkontakte mit einer Stromquelle verbunden, wobei ein ITO-Streifen positiv und ein Ca-Streifen negativ gepolt werden. Beim Anlegen einer genügend hohen Spannung, wird an dem entsprechenden Matrixelement eine Elektrofluoreszenz beobachtet.

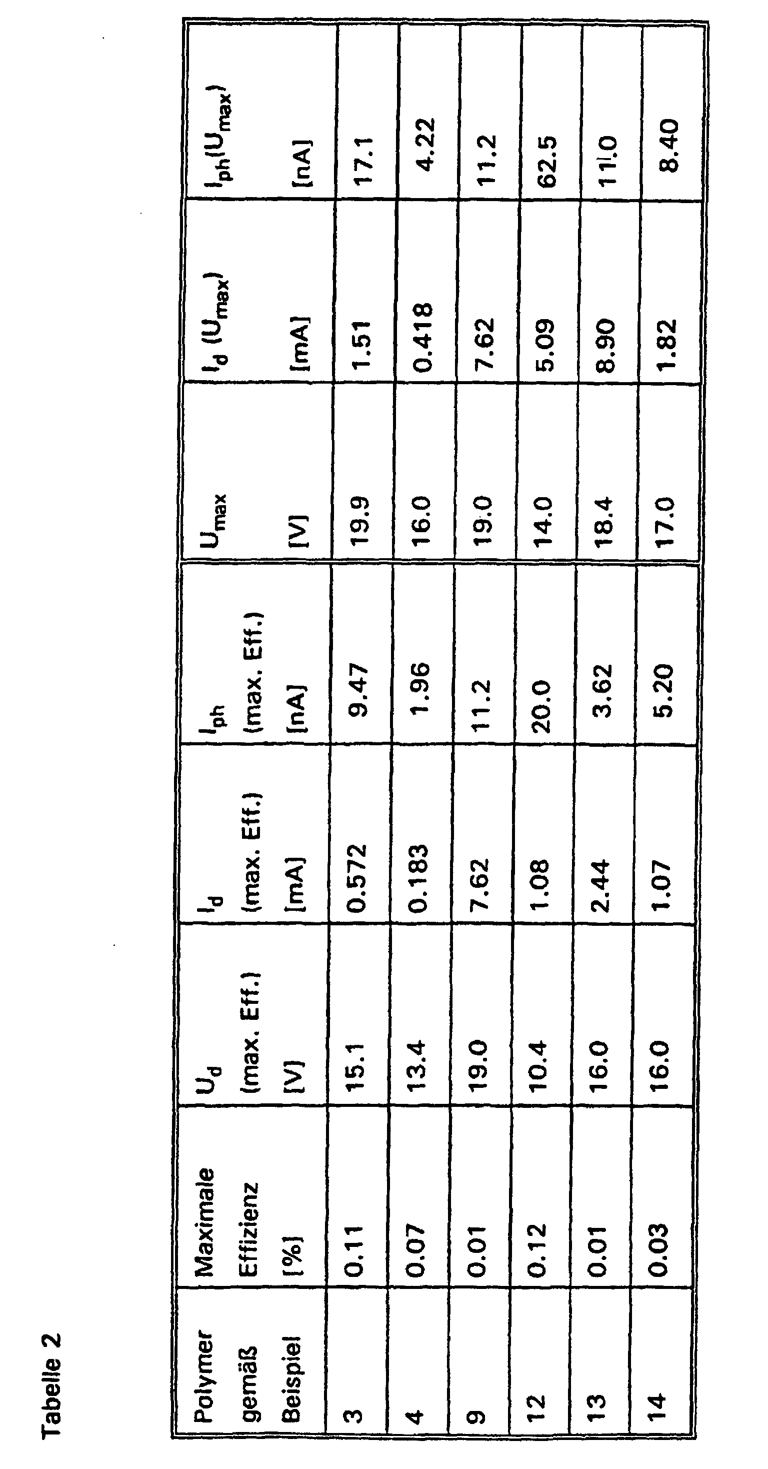

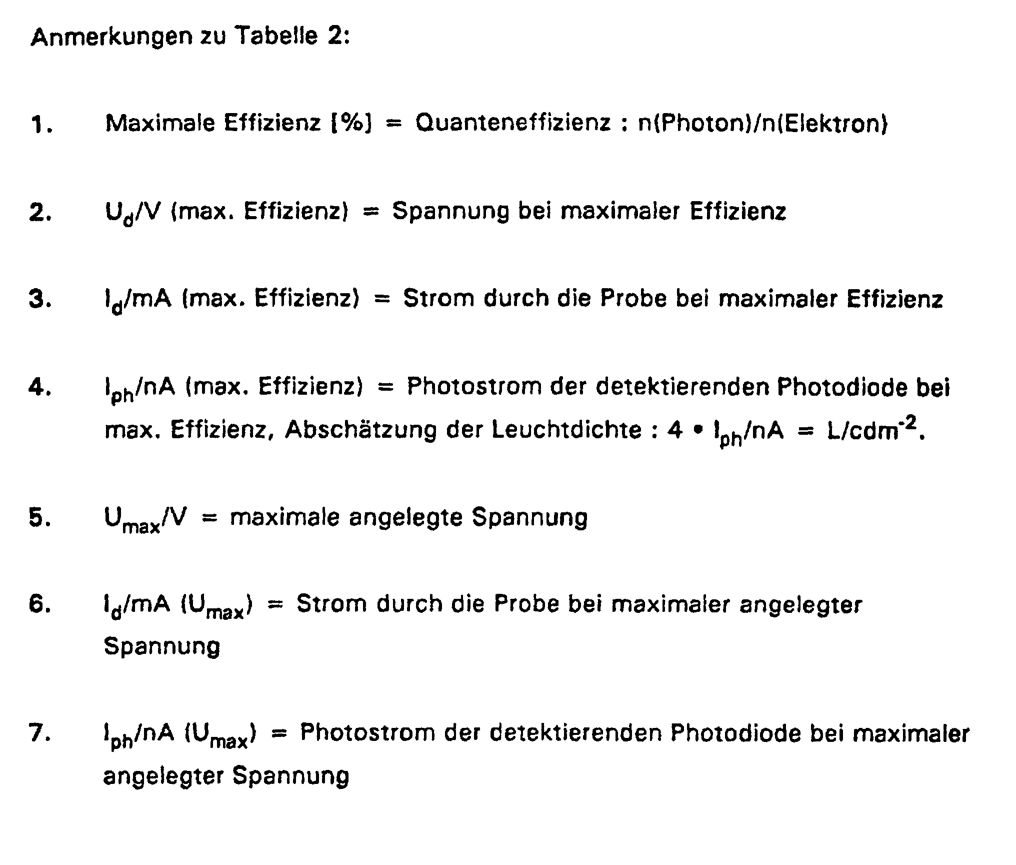

## Patentansprüche

1. Verwendung eines Polymers, enthaltend Struktureinheiten der allgemeinen Formel (I),
-[A¹-(A²)C = CH-A³-CH = C(A²)]- (I)
wobei A¹, A² und A³ gleich oder verschieden ein- und/oder mehrkernige, gegebenenfalls über eine oder mehrere, vorzugsweise eine Brücke verknüpfte, und/oder kondensierte Aryl- und/oder Heteroarylgruppen sind, die gegebenenfalls substituiert sein können, und wobei von A¹ und A³ je zwei und von A² je eine Bindung ausgeht,
als Elektrolumineszenzmaterial.

2. Verwendung nach Anspruch 1, **dadurch gekennzeichnet, daß** das Polymer, enthaltend Struktureinheiten der Formel (I), 2 bis 1000 Struktureinheiten aufweist.

3. Verwendung nach Anspruch 1 und/oder 2, **dadurch gekennzeichnet, daß** Symbole in der allgemeinen Formel (I) folgende Bedeutung haben:
A¹, A³: sind gleich oder verschieden 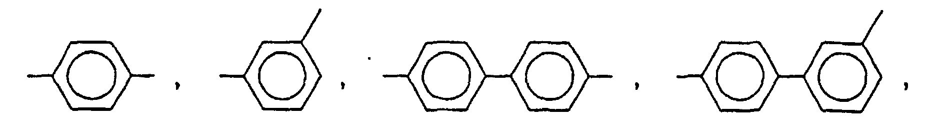 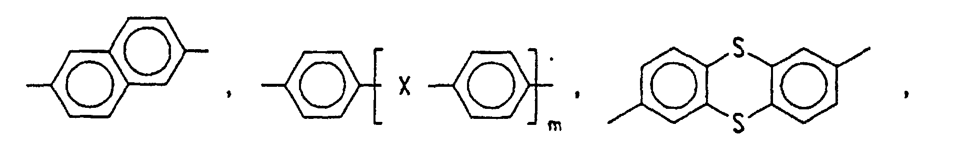 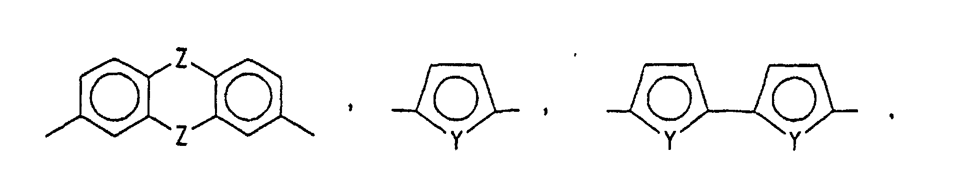 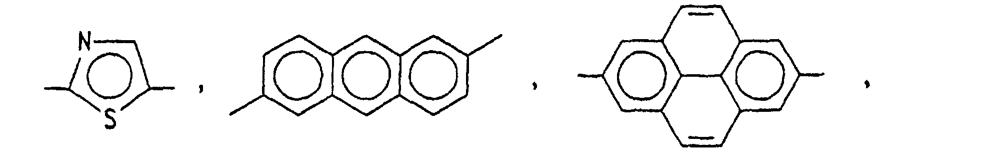 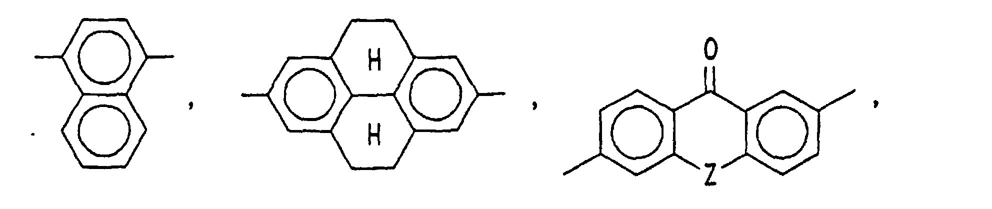 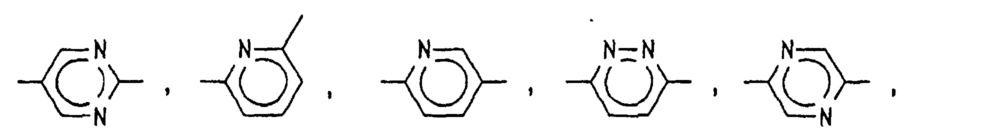 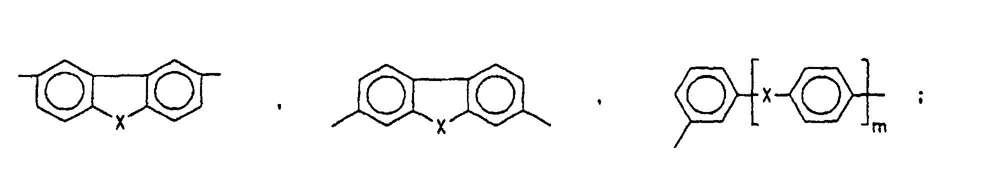 mit m = 1 bis 20, vorzugsweise 1, 2 oder 3, besonders bevorzugt 1,
vorzugsweise ist nur für A³ m > 1,
A²: hat, gleich oder verschieden von A¹ und A³, die gleichen Bedeutungen wie A¹ und A³, wobei von den beiden möglichen Bindungsstellen zum Polymer jeweils nur eine realisiert ist;
A¹, A² und A³ können dabei unabhängig voneinander mit einem oder mehreren Resten R substituiert sein;
X: eine Einfachbindung, -O-, -S-, -SO-, -SO₂-, -CRR-, -CR=CR-, -CH₂-CH₂oder -CHR-CHR-;
Y: -O-, -S- oder -NR'-;
Z: gleich oder verschieden -O- oder -S-;
R: gleich oder verschieden H, eine Alkylgruppe mit 1 bis 12 Kohlenstoffatomen, wobei auch eine oder zwei nicht benachbarte CH₂-Gruppen durch -O-, -S-, -CO-, -CO-O-, -0-OC- oder -Si(CH₃)₂- ersetzt sein können, -CF₃, -Ph, -O-Ph, -S-Ph, -SO-Ph, -SO₂-Ph, F, Cl, Br, I oder -CN;
R': H ,eine Alkylgruppe mit 1 bis 12 Kohlenstoffatomen oder -Ph.

4. Verwendung nach einem oder mehreren der vorhergehenden Ansprüche, **dadurch gekennzeichnet, daß** die Symbole in der Formel (I) folgende Bedeutungen haben:
A¹, A³: sind gleich oder verschieden 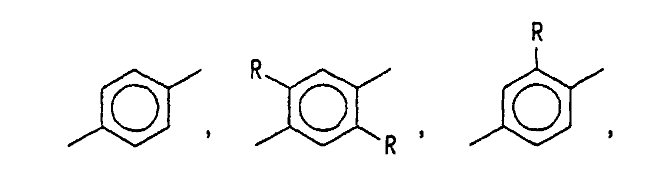 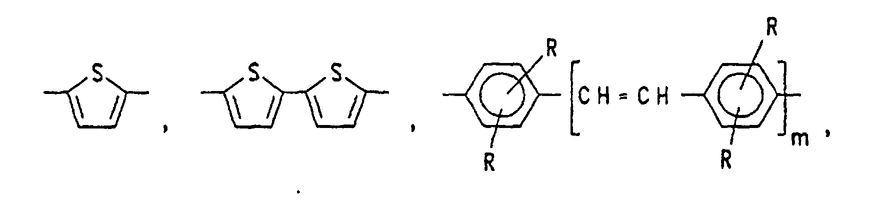 mit m = 1 bis 20, vorzugsweise 1, 2 oder 3, besonders bevorzugt 1, R ist vorzugsweise H, vorzugsweise ist nur für A³ m > 1, 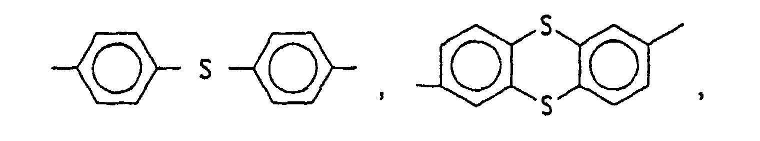
A²: hat, gleich oder verschieden von A¹ und A³, die gleichen Bedeutungen wie A¹ und A³, wobei von den beiden möglichen Bindungsstellen zum 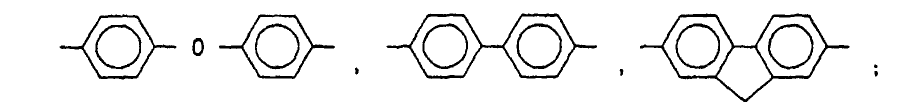 Polymer jeweils nur eine realisiert ist, oder ist

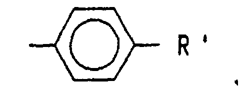

5. Verwendung nach einem oder mehreren der vorhergehenden Ansprüche, **dadurch gekennzeichnet, daß** das Polymer, enthaltend Struktureinheiten der Formel (I), aus der Gruppe (la) bis (lo) stammt, 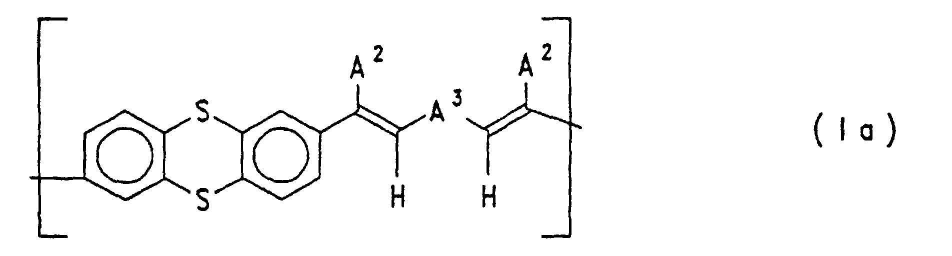 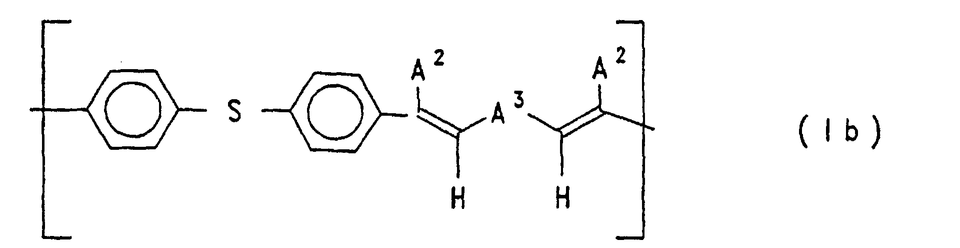 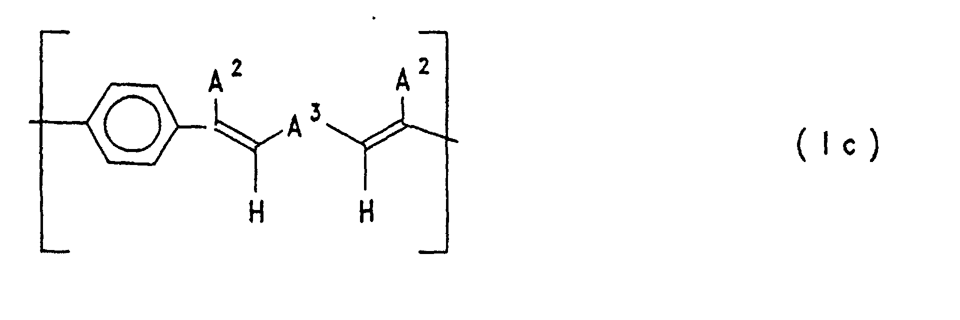 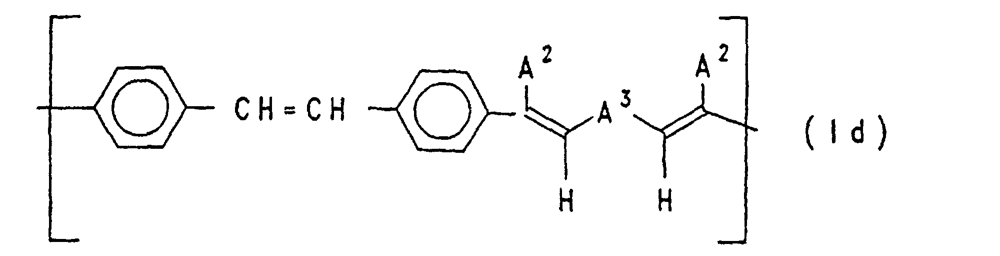 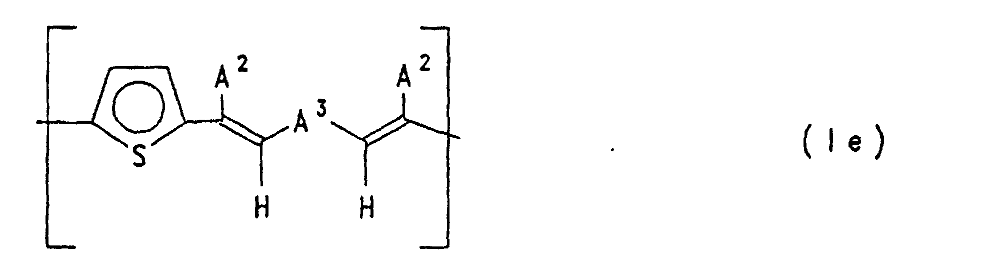 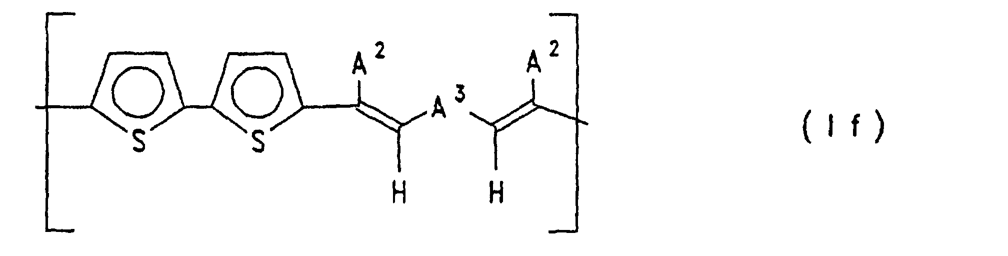 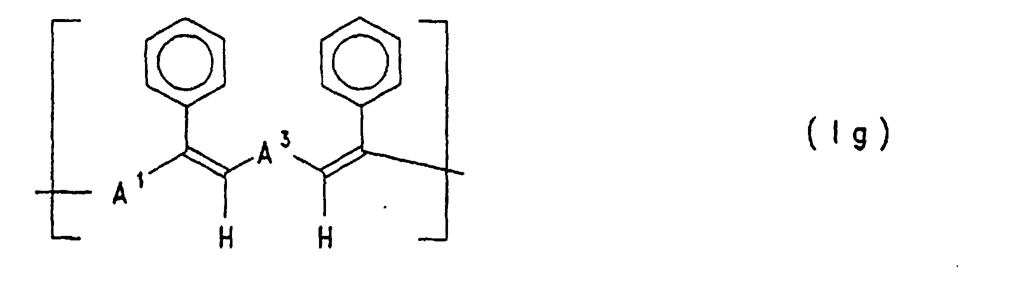 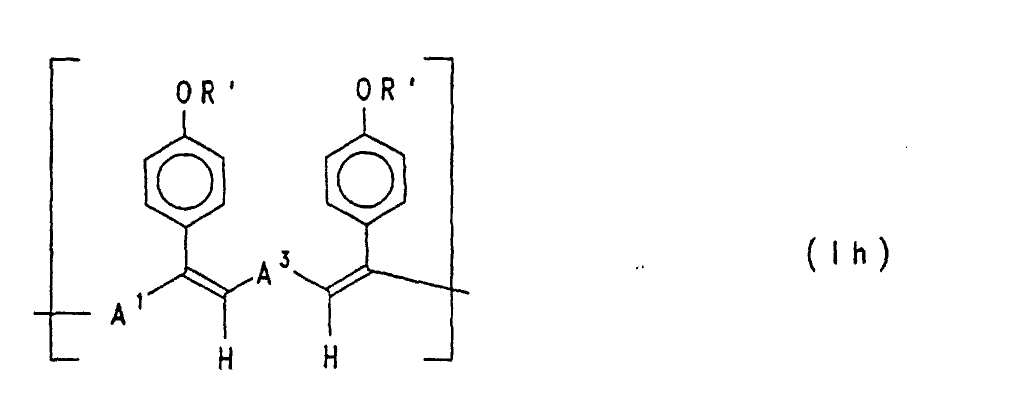 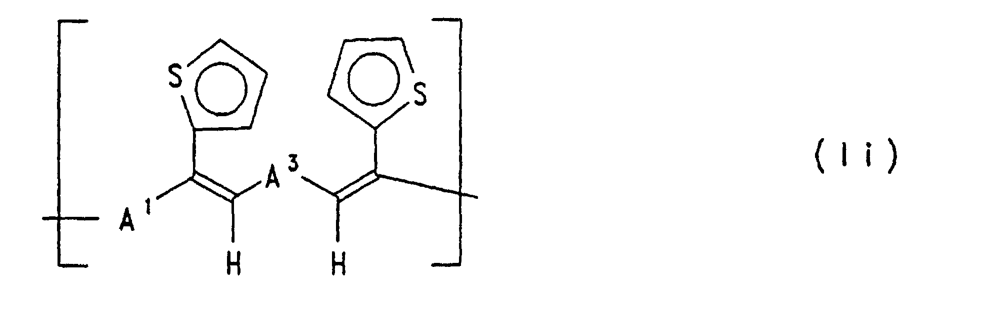 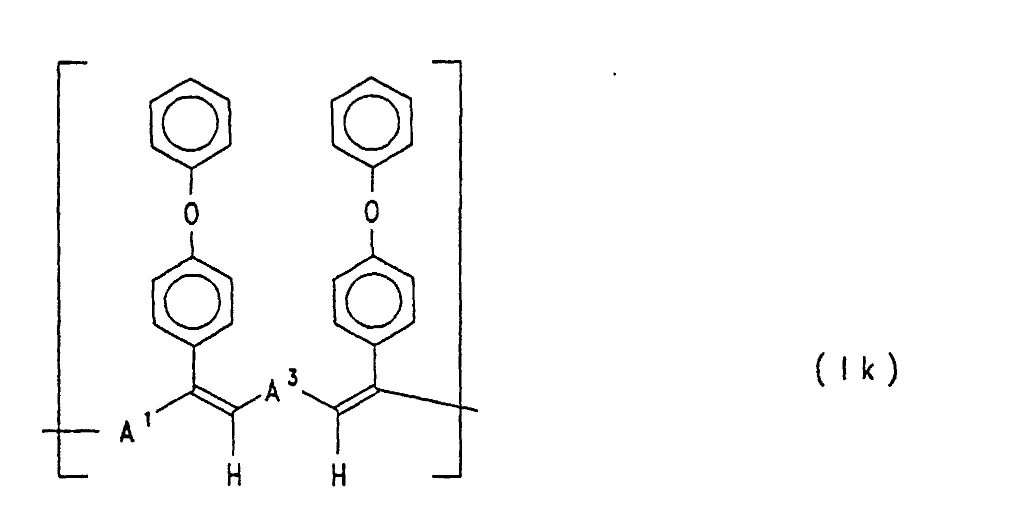 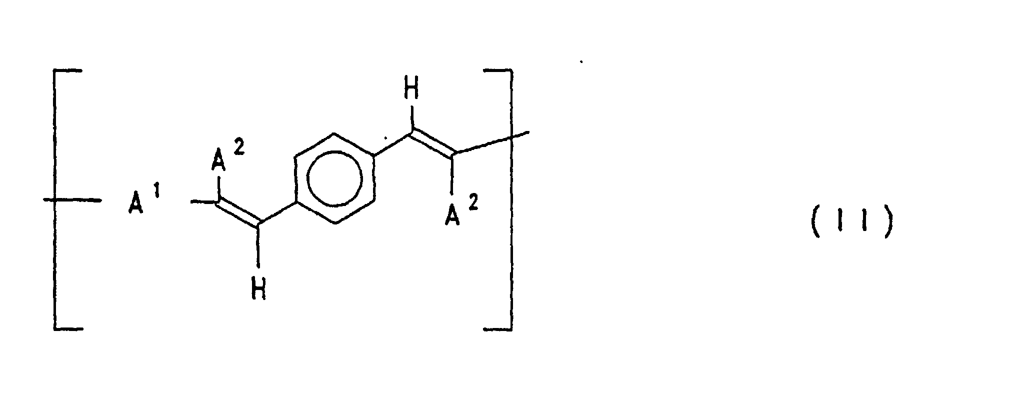 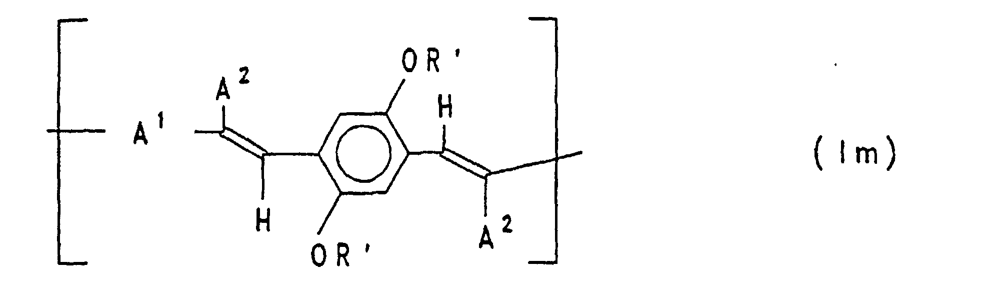 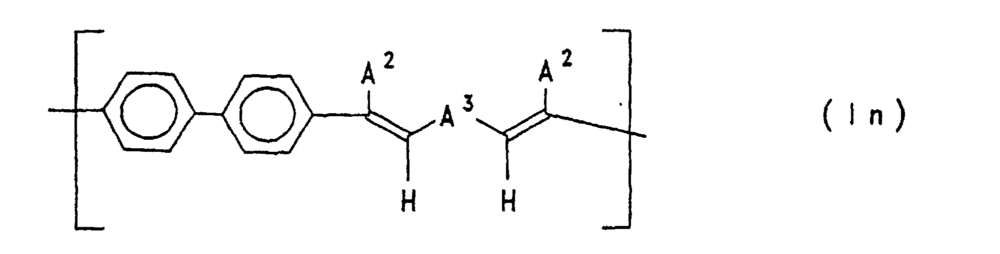 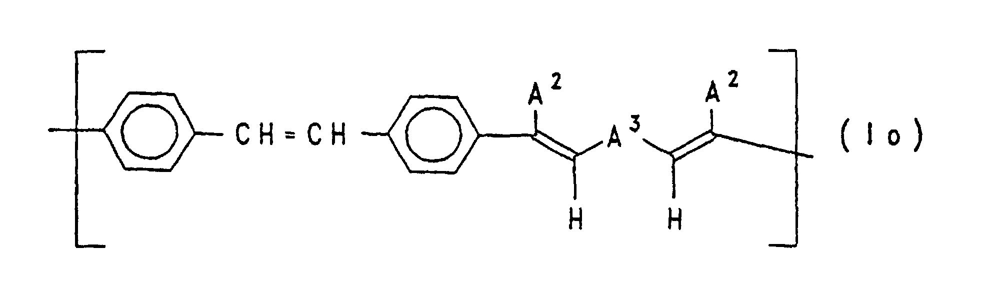 wobei A¹, A², A³ und R' die in Formel (I) angegebenen Bedeutungen haben.

6. Verwendung nach einem oder mehreren der vorhergehenden Ansprüche, **dadurch gekennzeichnet, daß** ein Copolymer, enthaltend Struktureinheiten der Formel (I), eingesetzt wird.

7. Elektrolumineszenzmaterial, enthaltend ein oder mehrere Polymere, enthaltend Struktureinheiten der Formel (I), gemäß einem oder mehreren der Ansprüche 1 bis 6.

8. Polymer, enthaltend Struktureinheiten der allgemeinen Formel (I),
-[A¹-(A²)C = CH-A³-CH = C(A²)]- (I)
wobei A¹, A² und A³ gleich oder verschieden ein- und/oder mehrkernige, gegebenenfalls über eine oder mehrere, vorzugsweise eine Brücke verknüpfte, und/oder kondensierte Aryl- und/oder Heteroarylgruppen sind, die gegebenenfalls substituiert sein können, und wobei von A¹ und A³ je zwei und von A² je eine Bindung ausgeht,
mit der Maßgabe, daß einer der Reste A¹, A² oder A³ ein Heterocyclus sein muß.

9. Elektrolumineszenzvorrichtung mit einer oder mehreren aktiven Schichten, **dadurch gekennzeichnet, daß** mindestens eine dieser aktiven Schichten ein Polymer gemäß einem oder mehreren der Ansprüche 1 bis 6 als Elektrolumineszenzmaterial enthält.

## Claims

1. Use of a polymer as an electroluminescent material, containing structural units of the general formula (I),
-[A¹-(A²)C = CH-A³-CH=C(A²)]- (I)
where A¹, A² and A³ are identical or different single or multi-cored aryl- and / or heteroaryl groups possibly cross-linked by one or more bridges but preferably one such bridge and / or condensed, which groups may be substituted, and whereby each of A¹ and A³ possesses two bonds and A² one bond.

2. Use in accordance with claim 1, **characterised in that** the polymer, containing structural units of the Formula (I) exhibits 2 to 1,000 structural units.

3. Use in accordance with claim 1 and / or 2, **characterised in that** symbols in the general formula (I) have the following meaning:
A¹, A³ are identical or different members of the following 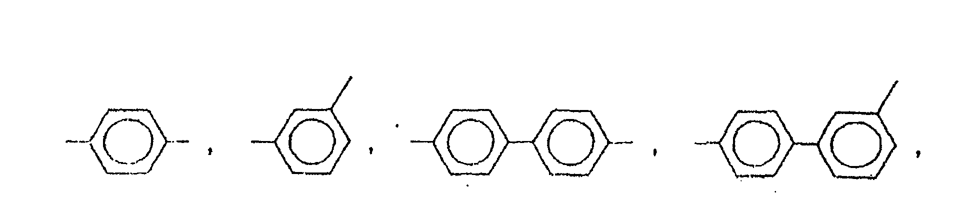 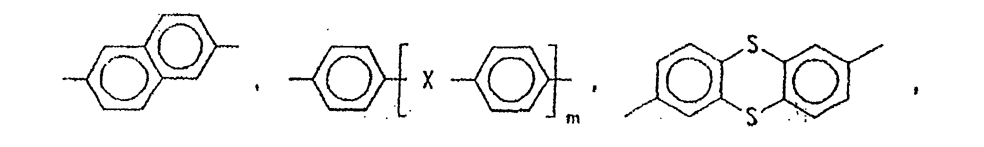 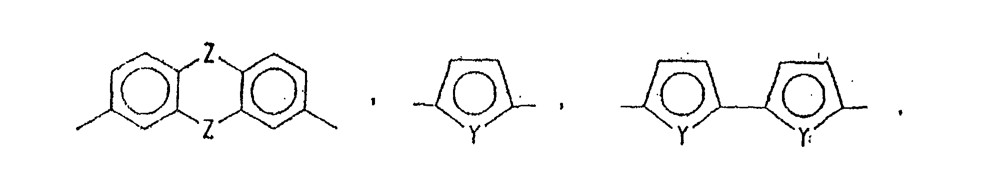 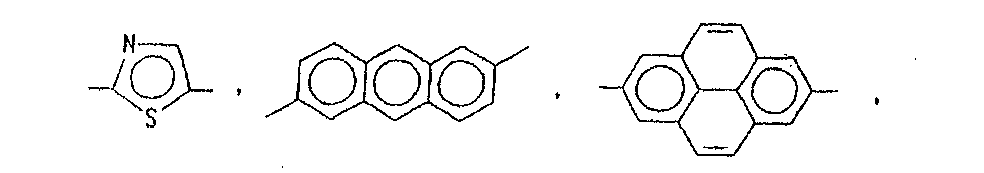 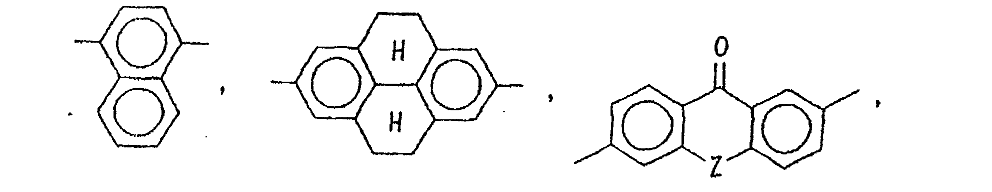 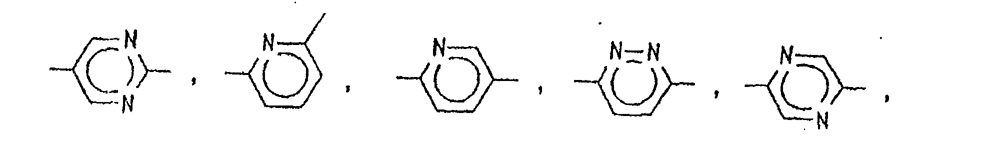 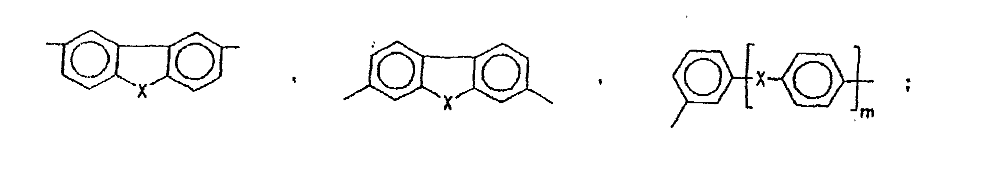 where m = 1 to 20, preferably 1, 2 or 3 with a special preference for 1 and preferably m > 1 only in the case of A^{3,}
A² : identically with or different from A¹ and A³ has the same meaning as A¹ and A³, where in each case only one of the two possible bonding points to the polymer is implemented;
A¹, A² and A³ may be, independently of one another, substituted by one or more residues R;
X: a single bond, -O-, -S-, -SO-, -SO₂-, - CRR-, -CR = CR-, -CH₂ - CH₂-, or -CHR-CHR-;
Y: -O-, -S- or -NR '-;
Z: identical or different -O- or -S-;
R: identical or different H, an alkyl group with 1 to 12 carbon atoms, where one or two non-adjacent CH₂- groups may be substituted by -O-, -S-, -CO-, -CO-O-, ,-O-OC- or - Si(CH₃)₂-, CF₃, -Ph, -O-Ph-, -S-Ph-, SO-Ph, -SO₂-Ph, F, Cl, Br or I or -CN:
R': H, an alkyl group with 1 to 12 carbon atoms or -Ph.

4. Use of one or more of the foregoing claims, **characterised in that** the symbols in Formula (I) have the following significance
A¹, A³ are identical or different members of the following 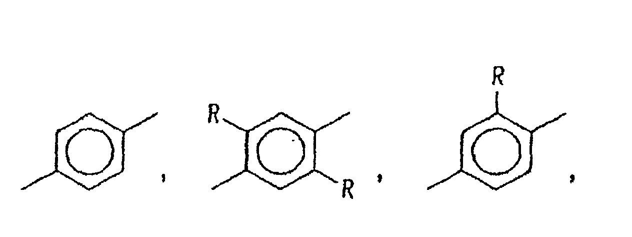 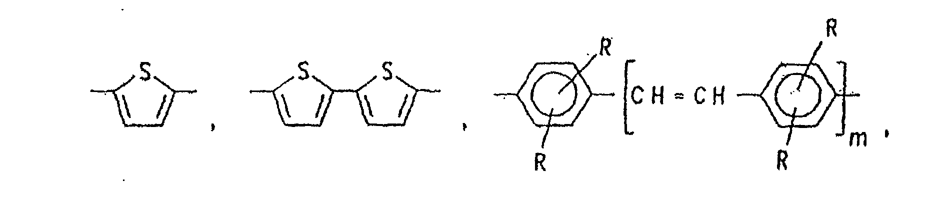 where m = 1 to 20, preferably 1,2 or 3 with a special preference for 1, R is preferably H and preferably m > 1 only in the case of A^{3,} 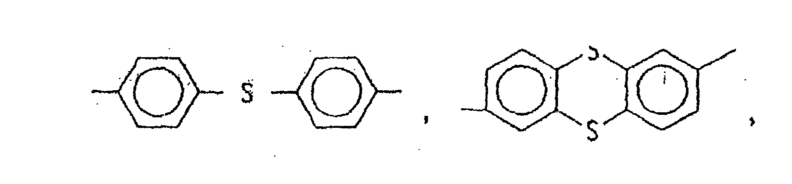
A² : identical with or different from A¹ and A³, has the same meaning as A¹ and A³, where in each case only one of the two possible bonding points to the polymer is implemented;

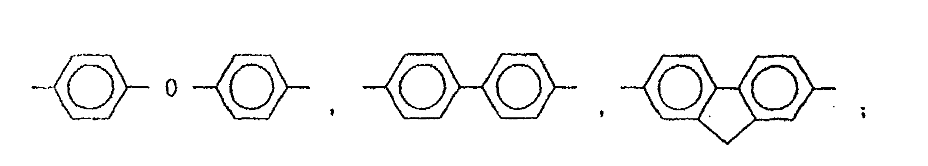

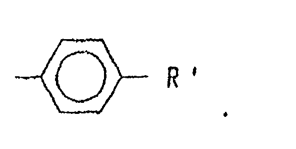

5. Use in accordance with one or more of the foregoing claims,
**characterised in that** the polymer containing structural units of Formula (1) originates from group (Ia) to (Io), 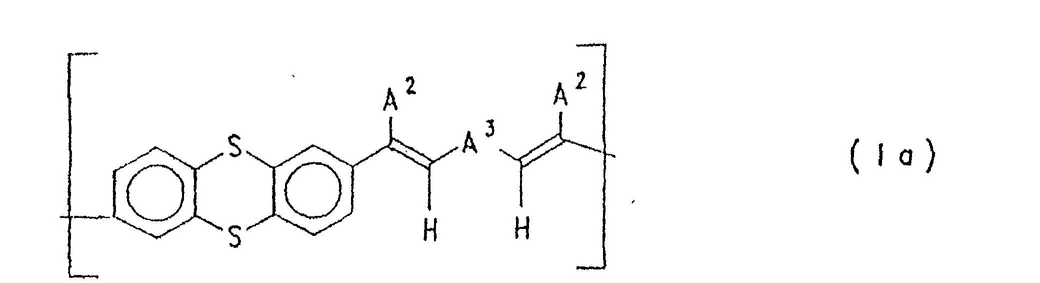 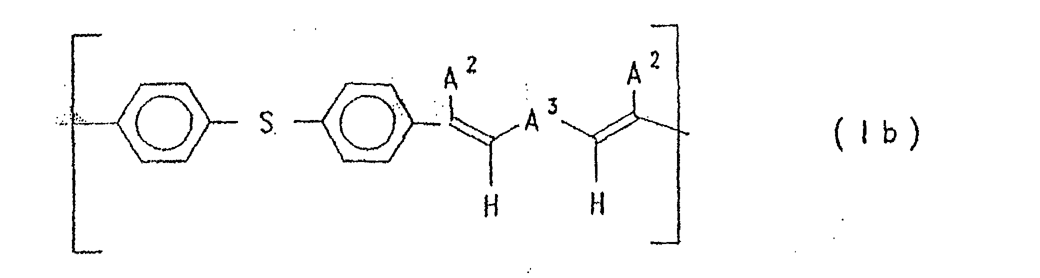 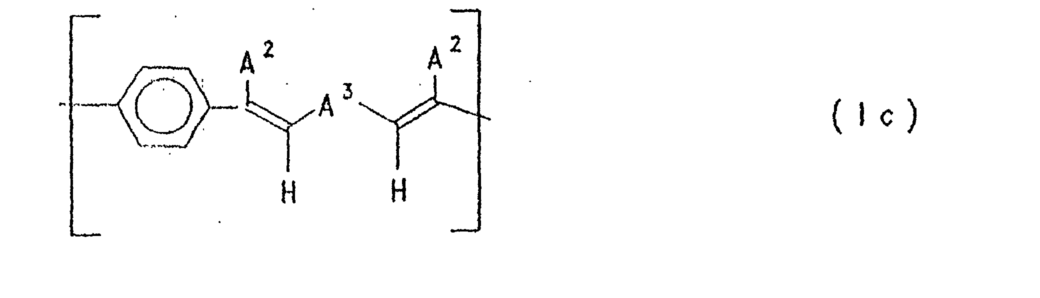 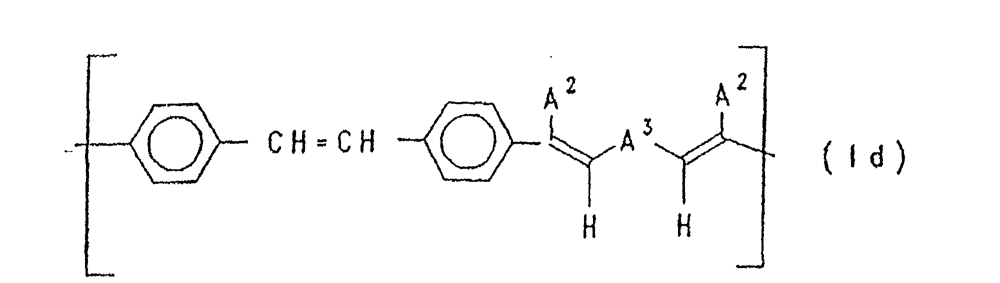 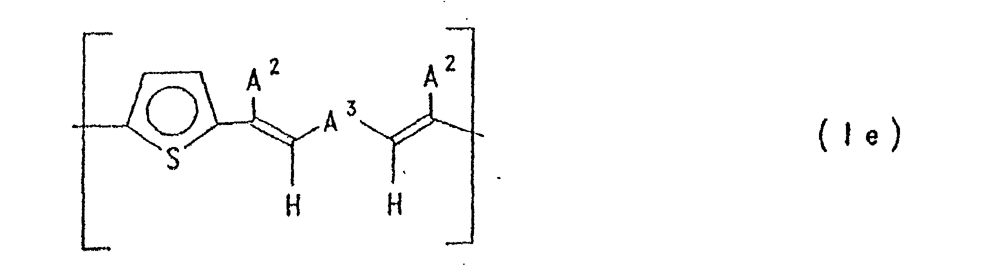 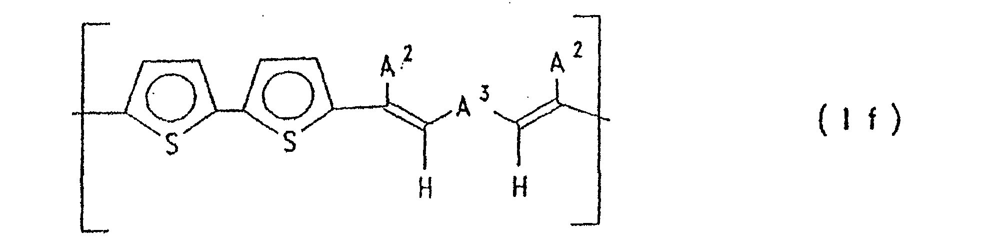 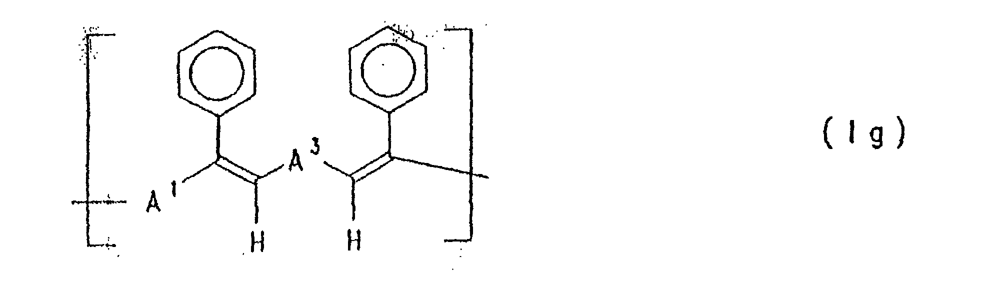 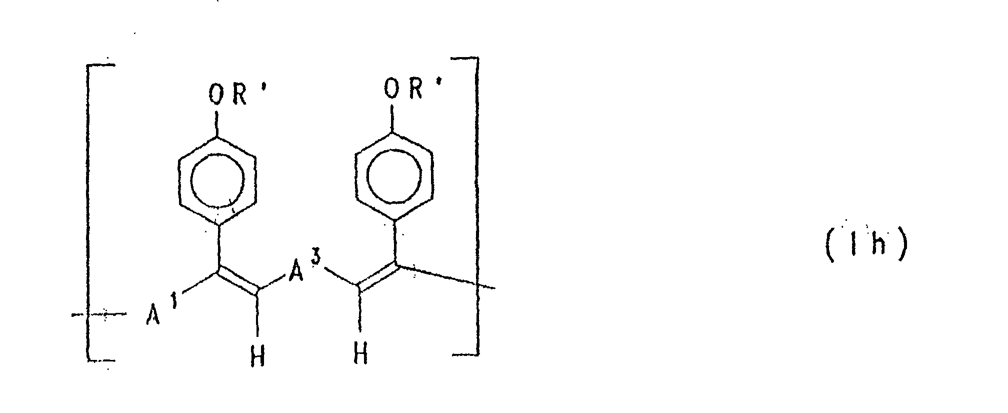 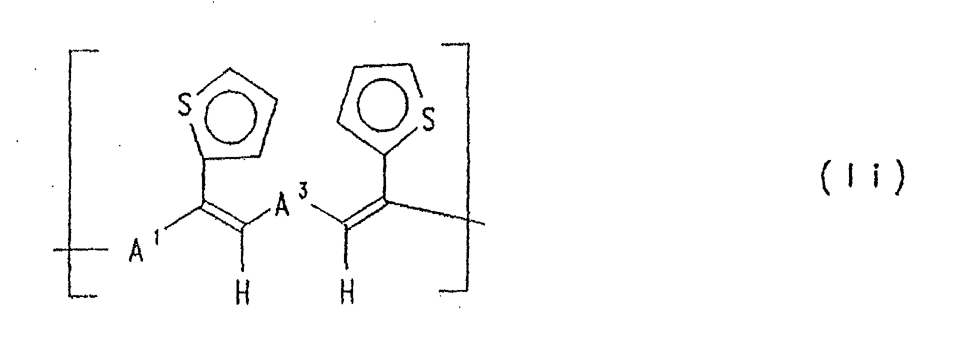 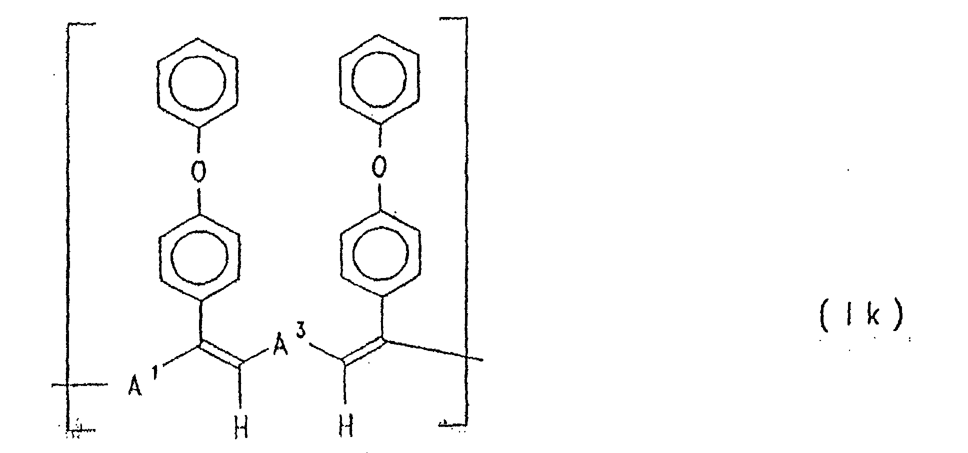 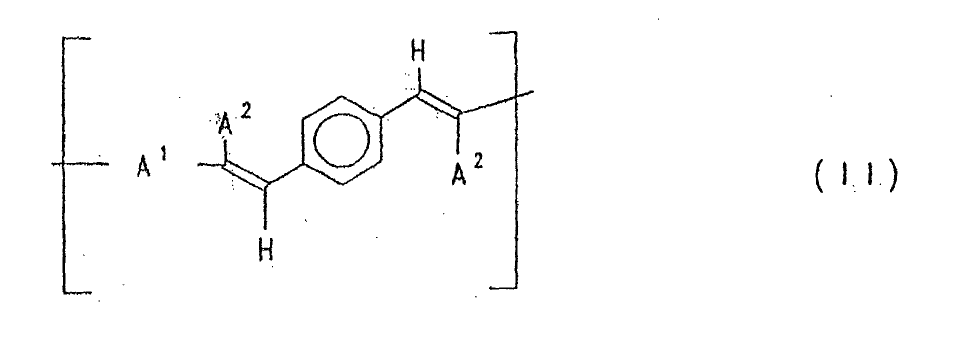 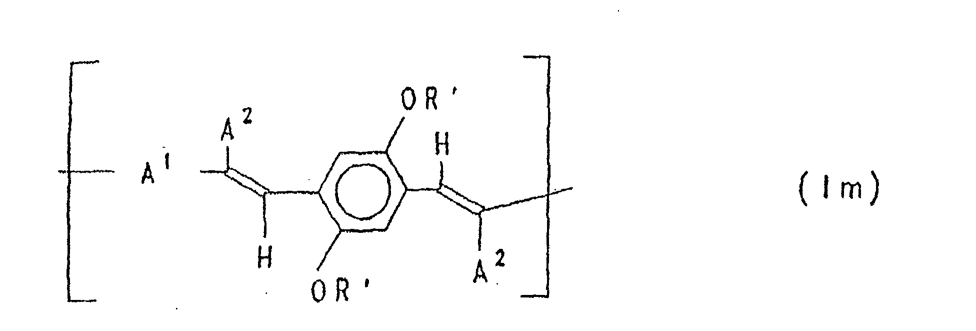 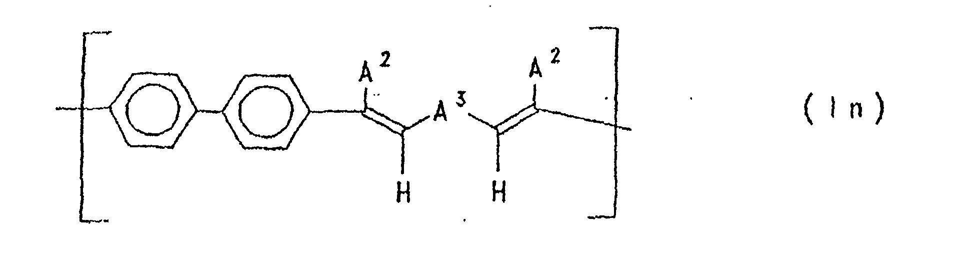 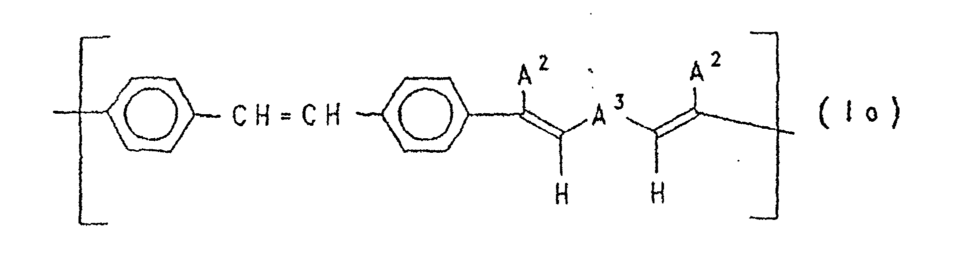 whereby A¹, A², A³ and R in Formula (I) have the indicated meanings.

6. Use in accordance with one or more of the foregoing claims,
**characterised in that** a copolymer containing structural units of Formula (I) is used.

7. Electroluminescent material, containing one or more polymers, containing structural units of Formula (I) in accordance with one or several of the claims 1 to 6

8. Polymer containing structural units of the General Formula (I),
-[A¹-(A²)C=CH-A³-CH=C(A²)]- (I)
where A¹, A² and A³ are identical or different single or multi-cored aryl- and / or heteroaryl groups possibly cross-linked by one or more bridges but preferably one such bridge and / or condensed, which groups may be substituted, and whereby each of A¹ and A³ possesses two bonds and A² one bond, with the requirement that one of the residues A¹, A² or A³ must be heterocyclic.

9. An electroluminescent device with one or more active layers, **characterised in that** at least one of these active layers contains a polymer as electroluminescent material in accordance with one or more of claims 1 to 6.

## Revendications

1. Utilisation d'un polymère contenant des unités de structure de formule générale (I)
-[A¹-(A²)C=CH-A³-CH=C(A²)]- (I)
où A¹, A² et A³ identiques ou différents représentent des groupes aryle et/ou hétéroaryle mono- et/ou polynucléaires, éventuellement reliés par un ou plusieurs, de préférence par un pont, et/ou condensés, qui peuvent éventuellement être substitués, chaque fois deux liaisons sont partantes à partir de A¹ et A³ et chaque fois une liaison est partante partir de A¹,
en tant que matière électroluminescente.

2. Utilisation selon la revendication 1, **caractérisée en ce que** le polymère contenant des unités de structure de formule (I), présente 2 à 1000 unités de structure.

3. Utilisation selon la revendication 1 et/ou 2, **caractérisée en ce que** les symboles dans la formule générale (I) possèdent la signification suivante :
A¹, A³ : sont identiques ou différents 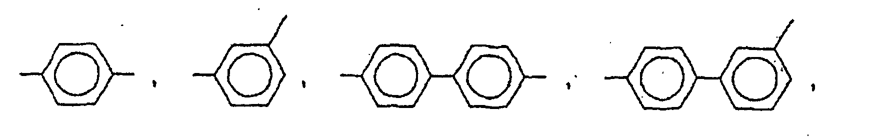 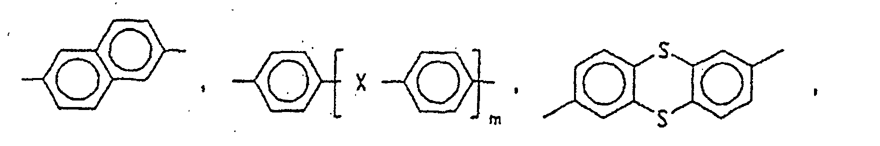 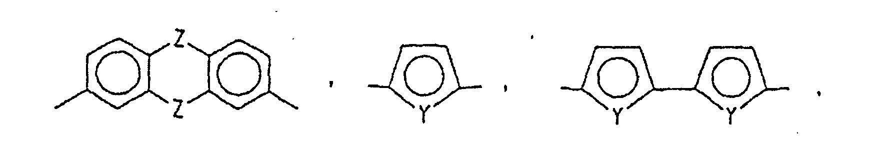 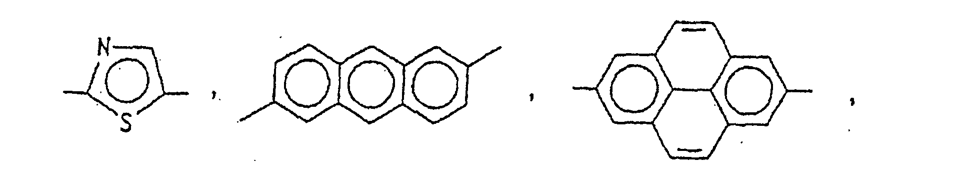 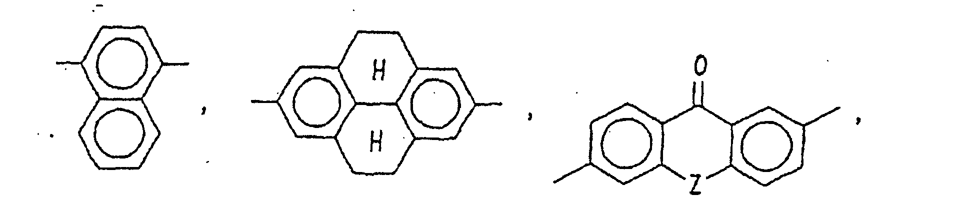 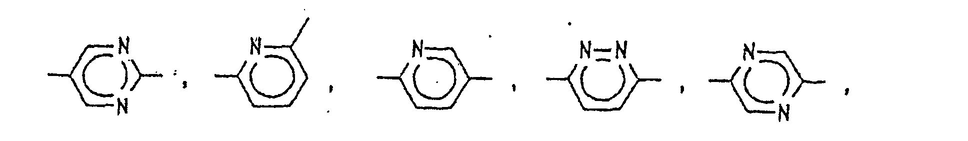 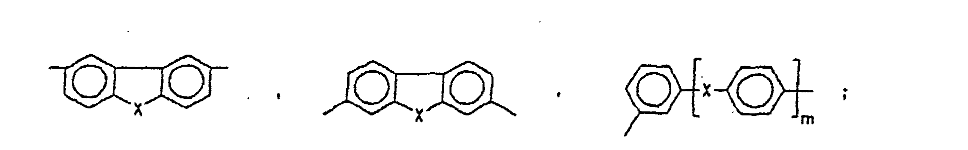 avec m = 1 à 20, de préférence 1, 2 ou 3, de manière particulièrement préférée 1,
de préférence m >1 uniquement pour A³,
A² possède, identique ou différent de A¹ et A³, les mêmes significations que A¹ et A³, des deux sites . de liaison possibles au polymère, une seule étant réalisée chaque fois ;
A¹, A² et A³ peuvent alors être, indépendamment les uns des autres, substitués par un ou plusieurs restes R ;
X représente une simple liaison, des groupes -O-, -S-, -SO-, -SO₂-, -CRR-, -CR=CR-, -CH₂-CH₂- ou -CHR-CHR- ;
Y représente des groupes -O-, -S- ou -NR'- ;
Z identiques ou différents représentent des groupes -O- ou -S- ;
R identiques ou différents représentent un atome d'hydrogène, un groupe alkyle présentant 1 à 12 atomes de carbone, un ou plusieurs groupes CH₂-non adjacents pouvant être remplacés par des groupes -O-, -S-, -CO-, -CO-O-, -O-OC- ou -Si(CH₃)₂-, représentent des groupes -CF₃, -Ph, -O-Ph, -S-Ph, -SO-Ph, -SO₂-Ph, F, Cl, Br, I ou CN ;
R' représente un atome d'hydrogène, un groupe alkyle présentant 1 à 12 atomes de carbone ou -Ph.

4. Utilisation selon une ou plusieurs des revendications précédentes, **caractérisée en ce que** les symboles à la formule (I) possèdent les significations suivantes :
A¹, A³ : sont identiques ou différents 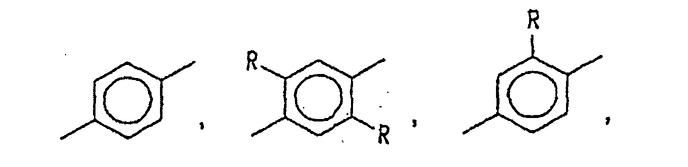 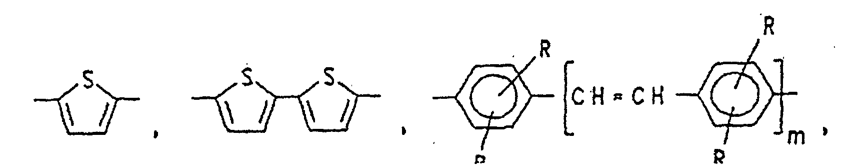 avec m = 1 à 20, de préférence 1, 2 ou 3, de manière particulièrement préférée 1, R représente de préférence un atome d'hydrogène,
de préférence m >1 uniquement pour A³, 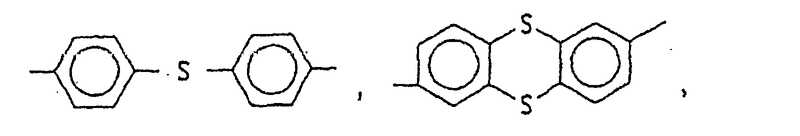
A² possède, identique ou différent de A¹ et A³, les mêmes significations que A¹ et A³, où des deux sites de liaison possibles au polymère 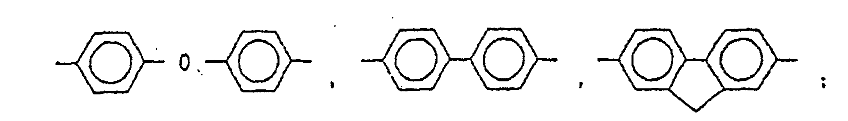 une seule étant réalisée chaque fois ou représente

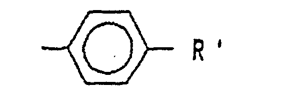

5. Utilisation selon une ou plusieurs des revendications précédentes **caractérisée en ce que** le polymère, contenant des unités de structure de formule (I) provient du groupe (Ia) à (Io) 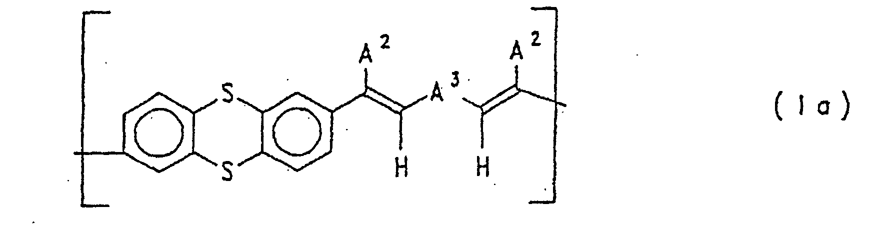 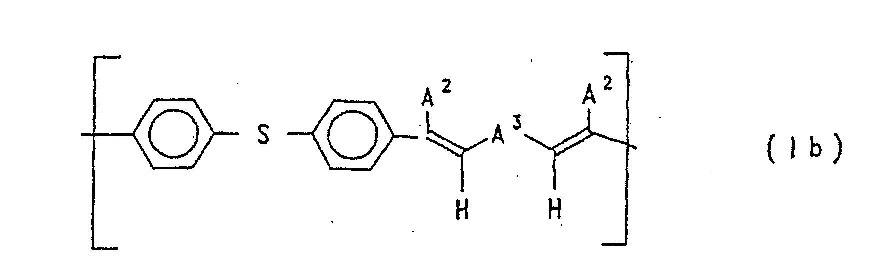 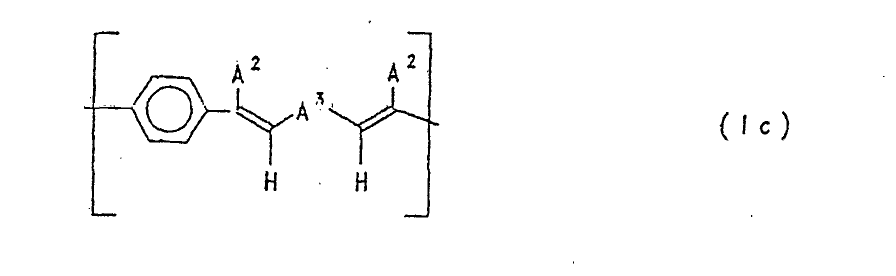 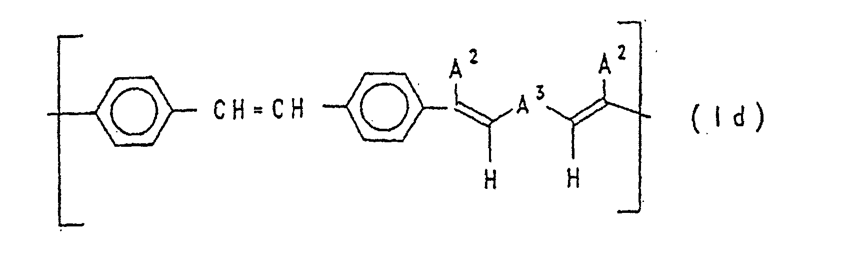 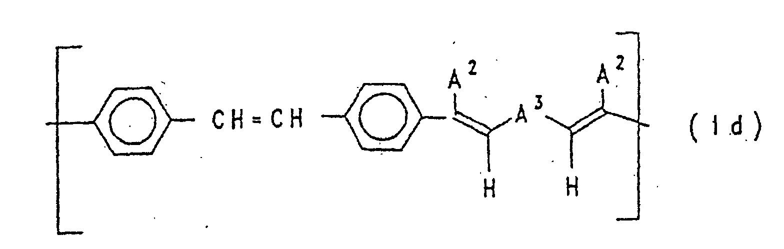 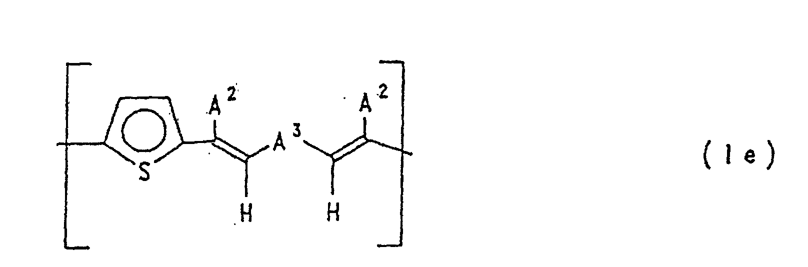 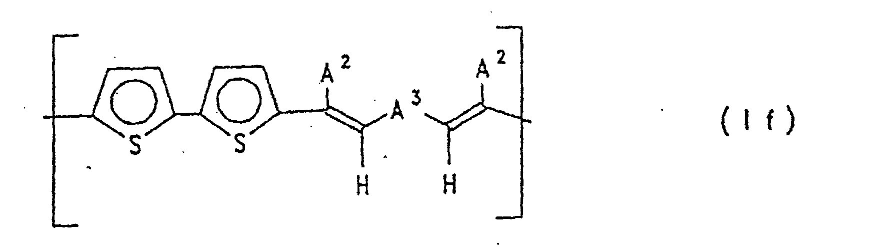 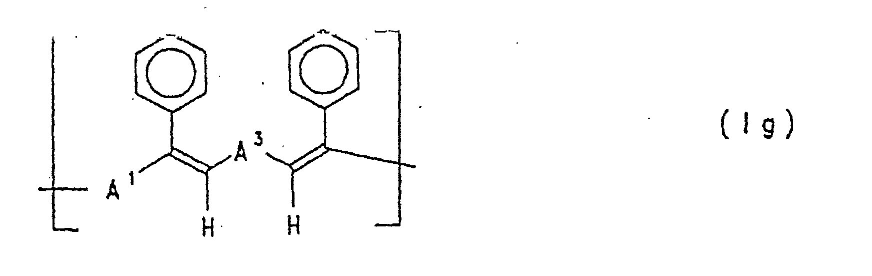 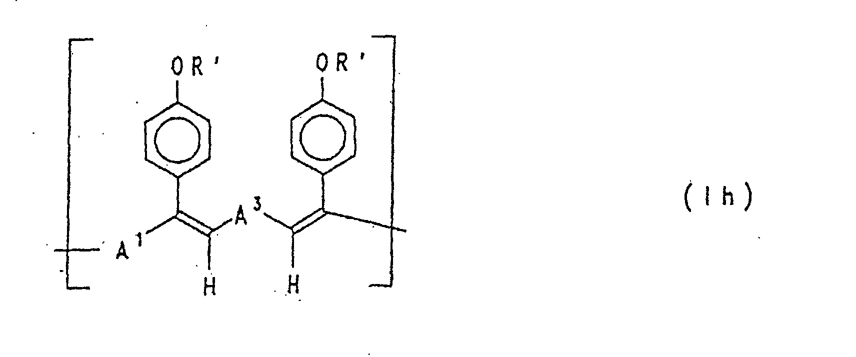 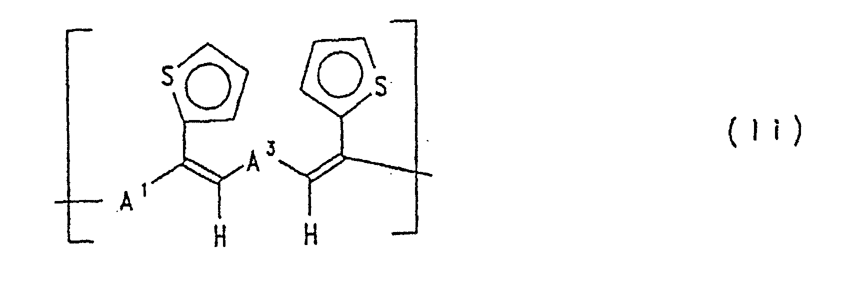 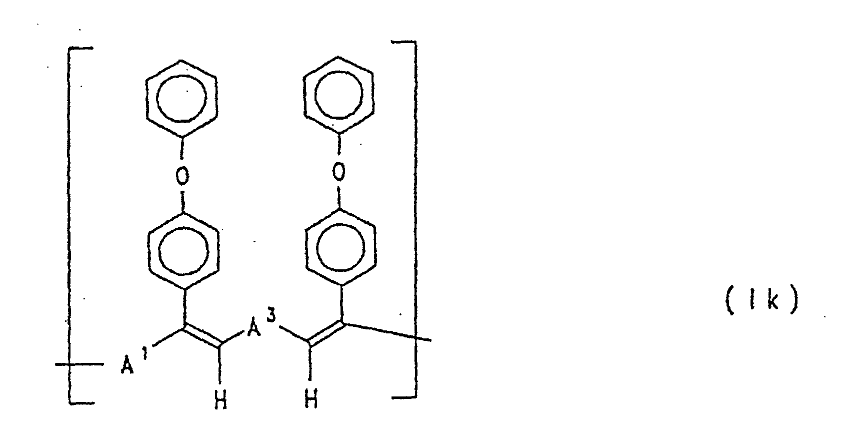 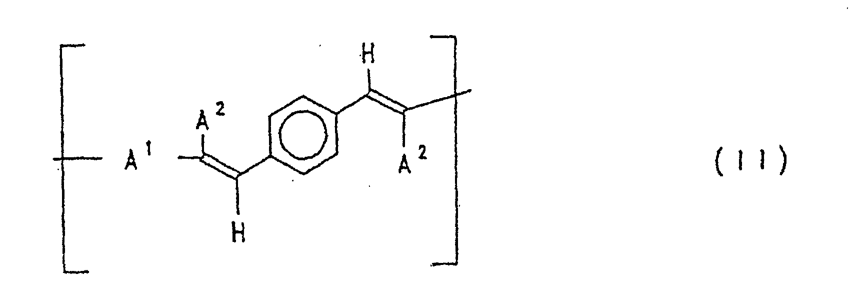 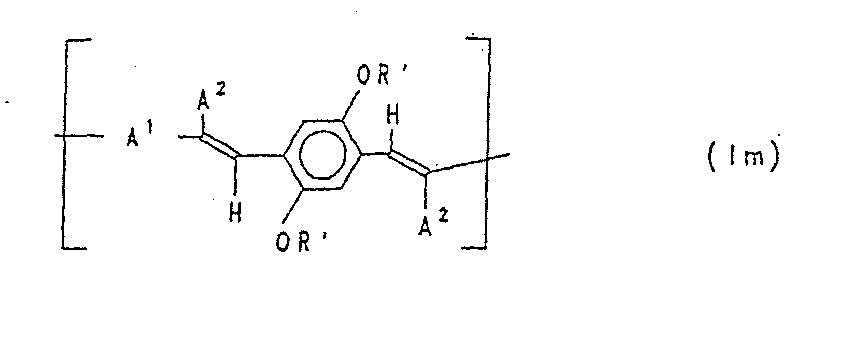 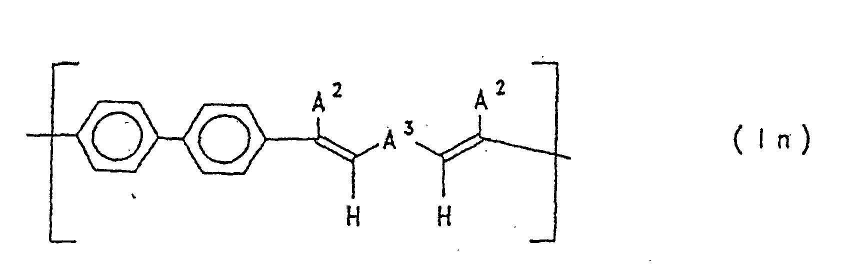 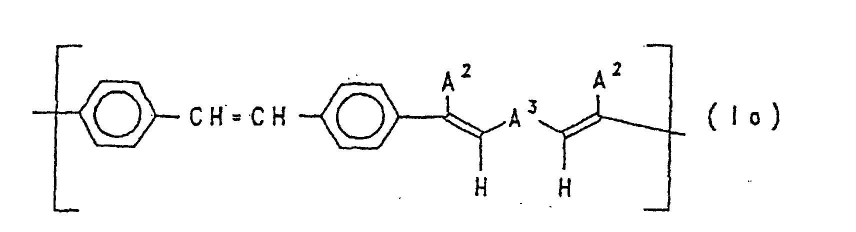 A¹, A², A³ et R' possèdent les significations données à la formule (I).

6. Utilisation selon une ou plusieurs des revendications précédentes, **caractérisée en ce qu'**on utilise un copolymère contenant des unités de structure de formule (I).

7. Matière électroluminescente renfermant un ou plusieurs polymères contenant des unités de structure de formule (I) selon une ou plusieurs des revendications 1 à 6.

8. Polymère contenant des unités de structure de formule générale (I)
-[A¹-(A²)C=CH-A³-CH=C(A²)]- (I)
où A¹, A² et A³ sont identiques ou différents représentant des groupes aryle et/ou hétéroaryle mono- et/ou polynucléaires, éventuellement reliés par un ou plusieurs, de préférence par un pont, et/ou condensés, qui peuvent éventuellement être substitués, chaque fois deux liaisons sont partantes à partir de A¹ et A³ et chaque fois une liaison est partante partir de A¹,
à condition que l'un des restes A¹, A² ou A³ soit un hétérocycle.

9. Dispositif électroluminescent présentant une ou plusieurs couches actives, **caractérisé en ce que** au moins une de ces couches actives contient un polymère selon une ou plusieurs des revendications 1 à 6 en tant que matière électroluminescente.
